(19) Europäisches Patentamt
European Patent Office
Office européen des brevets

(11) Veröffentlichungsnummer: **0 332 044 B1**

(12) **EUROPÄISCHE PATENTSCHRIFT**

(45) Veröffentlichungstag der Patentschrift: **16.06.93**

(21) Anmeldenummer: **89103609.7**

(22) Anmeldetag: **02.03.89**

(51) Int. Cl.5: **C07D 401/10**, C07D 403/10, C07D 413/10, C07D 417/10, C07D 417/14, G03C 1/685, G03C 1/72, G03F 7/105

# (54) 4,6-Bis-trichlormethyl-s-triazin-2-ylgruppen enthaltende heterocyclische Verbindungen, Verfahren zu ihrer Herstellung und lichtempfindliches Gemisch, das diese Verbindungen enthält.

(30) Priorität: **07.03.88 DE 3807381**

(43) Veröffentlichungstag der Anmeldung: **13.09.89 Patentblatt 89/37**

(45) Bekanntmachung des Hinweises auf die Patenterteilung: **16.06.93 Patentblatt 93/24**

(84) Benannte Vertragsstaaten: **CH DE FR GB LI NL**

(56) Entgegenhaltungen:
**EP-A- 0 135 348**
**EP-A- 0 135 863**
**EP-A- 0 137 452**
**US-A- 3 954 475**

(73) Patentinhaber: **HOECHST AKTIENGESELLSCHAFT**
**Postfach 80 03 20**
**W-6230 Frankfurt am Main 80(DE)**

(72) Erfinder: **Pawlowski, Georg, Dr.**
**Fritz-Kalle-Strasse 34**
**W-6200 Wiesbaden(DE)**
Erfinder: **Lutz, Heidrun**
**Martinstrasse 28-30**
**W-6500 Mainz(DE)**

**EP 0 332 044 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft 4,6-Bis-trichlormethyl-s-triazin-2-ylgruppen enthaltende heterocyclische aromatische Verbindungen, ein Verfahren zu ihrer Herstellung sowie ein lichtempfindliches Gemisch, das diese Verbindungen enthält.

Es ist bekannt, Trichlormethylgruppen enthaltende aromatische oder heterocyclische Verbindungen als Initiatoren für verschiedene photochemische Reaktionen einzusetzen.

Aus der DE-A 22 43 621 sind s-Triazine bekannt, die durch eine oder zwei Trichlormethylgruppen und eine chromophore Gruppe substituiert sind und als Photoinitiatoren in photopolymerisierbaren Gemischen sowie als Säurespender im Gemisch mit durch Säure spaltbaren Acetalen geeignet sind. Unter diesen Verbindungen sind auch solche, die im sichtbaren Bereich des elektromagnetischen Spektrums absorbieren und als Photoinitiatoren wirksam sind. Ihre Empfindlichkeit in diesem Bereich ist aber gering.

Ähnliche Verbindungen, in denen als chromophore Gruppe ein mindestens zweikerniger aromatischer Rest unmittelbar an den Triazinring gebunden ist, sind aus der DE-A 27 18 259 (= US-A 4 189 323) bekannt.

In der EP-A 137 452 sind ähnliche 4,6-Bis-trichlormethyl-s-triazine beschrieben, die in der 2-Stellung eine ggf. substituierte Styrylgruppe tragen. Die Absorptionsmaxima dieser Verbindungen liegen meistens im nahen UV-Bereich.

In der DE-A 28 51 472 sind lichtempfindliche Gemische beschrieben, die als Photoinitiatoren 2-Halogenmethyl-5-vinyl-1,3,4-oxadiazolderivate enthalten.

Aus den DE-A 30 21 590 und 30 21 599 sind mit Trichlormethylphenylgruppen substituierte Halogenoxazole bekannt, die ebenso wie alle zuvor genannten Verbindungen als Photoinitiatoren geeignet sind.

Schließlich sind aus den EP-A 135 348 und 135 863 1-Alkyl-2-carbonylmethylen-benzthiazole und ähnliche Heterocyclen bekannt, die an der Carbonylgruppe eine Trichlormethylphenylgruppe tragen. Auch diese Verbindungen haben ihre höchste Empfindlichkeit im nahen UV-Bereich.

Die bekannten Photoinitiatoren weisen die folgenden Nachteile auf:
Die Reaktionsbedingungen zur Herstellung vieler dieser Verbindungen sind verhältnismäßig drastisch, so daß die Ausbeute relativ gering ist und die Bildung von unerwünschten, schwer abtrennbaren Nebenprodukten begünstigt wird (z. B. DE-A 22 43 621, 27 18 259 oder 28 51 472).

Bei vielen bekannten Initiatoren erfordert die unzureichende Empfindlichkeit, daß verschiedene Initiatorsysteme miteinander kombiniert werden.

Grundsätzlich lassen sich mit den meisten vorstehend beschriebenen Initiatoren lichtempfindliche Gemische herstellen, deren Empfindlichkeit im nahen UV-Bereich gut ist, im sichtbaren Bereich aber nicht ausreicht. Diese Initiatoren weisen bei Belichtung mit gängigen Lasern, die sichtbares Licht relativ geringer Energie emittieren, keine oder nur eine geringe Initiatorwirkung auf. Darüber hinaus hat es sich erwiesen, daß gerade die empfindlichsten der bekannten Initiatoren keine den Anforderungen der Praxis entsprechende Lagerfähigkeit in lichtempfindlichen Gemischen, insbesondere im Kontakt mit Kupferoberflächen aufweisen.

Da aber in der Zukunft mit einem verstärkten Einsatz von Lasern, insbesondere Argon- oder Krypton-Lasern, die zwischen 450 und 650 nm emittieren und als Schreiblaser Verwendung finden, zu rechnen ist, bedarf es der Bereitstellung hochlichtempfindlicher Initiatoren und Gemische, die bei diesen Anwendungen ausreichende Empfindlichkeiten aufweisen.

Aufgabe der vorliegenden Erfindung ist es, neue lichtempfindliche Verbindungen bereitzustellen, die sich in verschiedenartigen lichtempfindlichen Materialien einsetzen lassen, die relativ einfach zugänglich sind und bei ihrem Einsatz eine große Breite von Variationsmöglichkeiten bieten, um den Bedürfnissen auf den verschiedenen Anwendungsgebieten jeweils optimal angepaßt werden zu können. Insbesondere ist es die Aufgabe der vorliegenden Erfindung, lichtempfindliche Verbindungen bereitzustellen, die im sichtbaren Spektralbereich hohe Empfindlichkeiten aufweisen, so daß ihre Aktivität bereits durch schwache, sichtbares Licht bei Wellenlängen oberhalb 400 nm, insbesondere bei 488 und 514 nm, emittierende Lichtquellen angeregt wird. Zusätzlich sollen die Verbindungen bei Einsatz in lichtempfindlichen Gemischen für die Reprographie, z. B. in Druckplatten, bereits nach der Bestrahlung einen deutlich sichtbaren Bildkontrast in der lichtempfindlichen Schicht ergeben. Ferner sollen die lichtempfindlichen Gemische, die die neuen Initiatoren enthalten, unabhängig vom Material des Schichtträgers, auf dem sie sich befinden, eine hohe Lagerstabilität aufweisen.

Die Aufgabe wird gelöst durch Verbindungen der allgemeinen Formel I

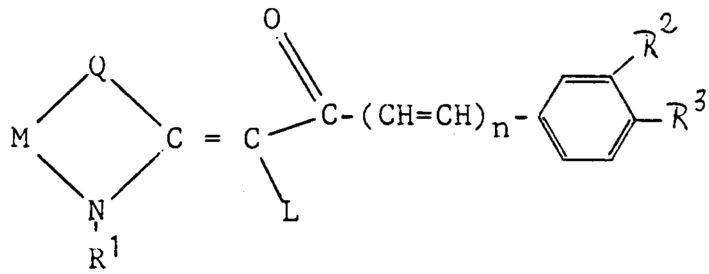

(I)

worin

L ein Wasserstoffatom oder einen Substituenten der Formel

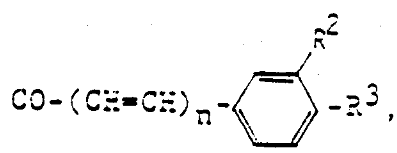

M einen 1,1-, 1,2- oder 1,3-Alkylenrest oder einen 1,2- oder 1,3-Alkenylenrest, die gegebenenfalls durch Halogenatome, Carboxy-, Carbonyl-, Alkoxy-, Alkyl- oder Arylgruppen substituiert sind; einen 1,2-Phenylenrest, der gegebenenfalls durch Halogenatome, Carboxy-, Sulfonsäure-, Nitro-, Cyan-, Carbonyl-, Alkyl-, Aryl-, Alkoxy-, Trifluormethyl- oder Alkoxycarbonylalkylgruppen substituiert ist, einen Pyridylen- oder Naphthylenrest,

Q ein Schwefel-, Selen- oder Sauerstoffatom, eine Dialkylmethylengruppe mit 3 bis 13 Kohlenstoffatomen, eine 1,2-Alkenylengruppe, die ggf. durch 1 oder 2 Alkyl- oder Arylreste, Chloratome, Alkoxygruppen oder Alkoxy-carbonylgruppen substituiert ist, einen gegebenenfalls durch Chloratome, Alkoxy- oder Alkoxycarbonylgruppen substituierten 1,2-Phenylenrest oder eine Gruppe N-$R^1$,

wobei M + Q zusammen 3 oder 4 Ringglieder bilden,

$R^1$ einen Alkyl- oder Alkoxyalkylrest mit 1 bis 10 Kohlenstoffatomen, einen Benzyl-, Chlorbenzyl-, Tolylmethyl-, Phenethyl- oder Cyclohexylrest,

$R^2$ und $R^3$ voneinander verschieden sind und entweder ein Wasserstoffatom oder eine 4,6-Bis-trichlormethyl-s-triazin-2-yl-gruppe bedeuten und

n = 0 oder 1 ist.

Erfindungsgemäß wird ferner ein lichtempfindliches Gemisch vorgeschlagen, das eine lichtempfindliche organische Verbindung (a) mit mindestens einem 4,6-Bis-trichlormethyl-s-triazin-2-ylsubstituenten und eine Verbindung (b), die mit dem Lichtreaktionsprodukt der Verbindung (a) unter Ausbildung eines Produktes zu reagieren vermag, das eine von (b) unterschiedliche Lichtabsorption, Klebrigkeit oder Löslichkeit in einem Entwickler aufweist.

Das erfindungsgemäße Gemisch ist dadurch gekennzeichnet, daß die Verbindung (a) eine Verbindung gemäß der oben angegebenen Formel I ist.

Die erfindungsgemäßen Verbindungen bilden unter Einwirkung von aktinischer Strahlung freie Radikale, die zur Einleitung chemischer Reaktionen, insbesondere von radikalisch initiierten Polymerisationen, befähigt sind. Sie bilden ferner bei Bestrahlung Halogenwasserstoff, durch den säurekatalysierte Reaktionen, z. B. die Spaltung von Acetalbindungen, oder Salzbildungen, z. B. Farbumschläge von Indikatorfarbstoffen, in Gang gesetzt werden können.

In der Formel I sind L und $R^2$ vorzugsweise Wasserstoffatome. M ist bevorzugt ein 1,2-Phenylenrest, der gegebenenfalls durch Halogenatome, Carboxy-, Sulfonsäure-, Nitro-, Cyan-, Carbonyl-, Alkyl-, Aryl-, Alkoxy-, Trifluormethyl- oder Alkoxycarbonylalkylgruppen substituiert sein kann, aber bevorzugt unsubstituiert ist.

Q ist bevorzugt ein Schwefel- oder Selenatom, eine Gruppe $NR^1$ oder eine Dialkylmethylengruppe mit 3 bis 13, vorzugsweise 3 bis 7, insbesondere 3 Kohlenstoffatomen. Wenn Q eine Dialkylmethylengruppe ist, können die Alkylgruppen unter Ausbildung eines 5- oder 6-gliedrigen Rings miteinander verbunden sein. Besonders bevorzugt ist Q = S oder Se, insbesondere als Bestandteil eines fünfgliedrigen Rings.

Wenn $R^1$ ein Alkyl- oder Alkoxyalkylrest ist, kann dieser bevorzugt 1 - 6 Kohlenstoffatome enthalten. Er kann geradkettig oder verzweigt oder ggf. zu einem cycloaliphatischen Rest, z. B. einem Cyclohexylrest, ringgeschlossen sein. $R^1$ ist besonders bevorzugt ein Alkylrest mit 1-6 Kohlenstoffatomen.

Im allgemeinen werden Verbindungen mit $n = 0$ bevorzugt.

Die erfindungsgemäßen Verbindungen lassen sich vorteilhaft in Analogie zu bekannten Verfahren [z. B. A. Mistr, V. Laznicka u. M. Vavra, Coll. Czech. Chem. Commun. 36, 150 (1971)] aus einer Methylenverbindung der Formel II oder dem entsprechenden Iminiumsalz der Formel III und einem Carbonsäurehalogenid der Formel IV herstellen:

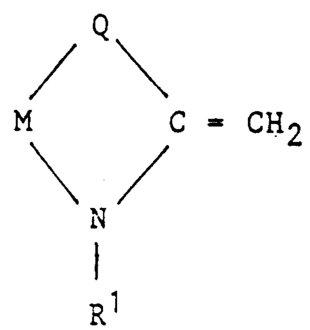

(II)

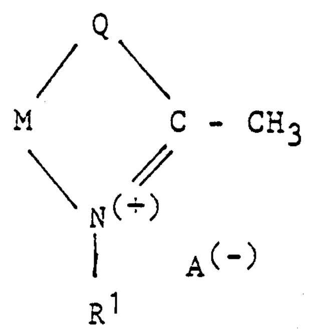

(III)

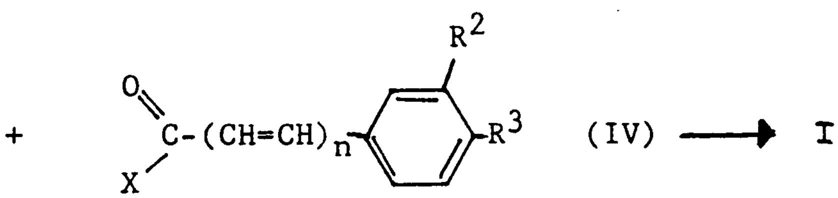

(IV) I

wobei A ein anorganisches Anion, bevorzugt ein Halogenidanion, Tetrafluoroboratanion, ein Perchloratanion oder ein organisches Anion, bevorzugt ein Sulfonatanion oder ein Alkylsulfatanion, X ein Halogenatom bedeutet und die restlichen Symbole die oben angegebene Bedeutung haben.

Die Umsetzung findet bevorzugt unter dem Einfluß von Stickstoffbasen, z. B. Triethylamin, Dimethylbenzylamin, Diethylbenzylamin, N-Ethyldicyclohexylamin, N-Ethylpiperidin, N-Methylpiperidin, N-Methylmorpholin, N-Ethylmorpholin, N-Ethylpyrrolidon, 1,8-Diaza-bicyclo[5,4,0]undec-7-en, 1,4-Diaza-bicyclo[2,2,2]octan oder Pyridin, statt, wobei die Base selbst als Lösungsmittel dienen kann oder ein inertes organisches Lösungsmittel zugesetzt wird, z. B. Benzol, Toluol, Dimethylformamid, Tetrahydrofuran, Diethylether, Diisopropylether oder Methylenchlorid. Die Umsetzung wird vorteilhaft bei Temperaturen zwischen 0° und 100°C durchgeführt, wobei die Menge des Carbonsäurehalogenids im allgemeinen zwischen 1 und 4, für die Herstellung von Produkten mit L = H bevorzugt zwischen 1 und 1,5 und für die Herstellung von disubstituierten Produkten zwischen 2 und 3 mol je mol II bzw. III liegt.

Die Säurehalogenide der Formel IV können durch Cotrimerisierung von Trichloracetonitril mit 3- oder 4-Cyanobenzoyl- oder -cinnamoylchlorid unter Einwirkung von HCl-Gas in Gegenwart einer Lewis-Säure

hergestellt werden. Bevorzugt wird aber zunächst die 3- oder 4-(4,6-Bis-trichlormethyl-s-triazin-2-yl)-benzoe- oder -zimtsäure durch entsprechende Cotrimerisierung mit 3- oder 4-Cyanobenzoe- oder -zimtsäuremethylester, Umesterung der erhaltenen Ester mit Trichloressigsäure in Gegenwart einer starken Mineralsäure und Abdestillieren des gebildeten Trichloressigsäuremethylesters hergestellt und die erhaltene Säure z. B. mit Thionylchlorid in das Säurechlorid übergeführt.

Die Herstellung dieser Verbindungen ist im einzelnen in der gleichzeitig eingereichten Patentanmeldung P 38 07 378.1 beschrieben.

Die Verbindungen der Formeln II und III sind teils handelsüblich, teils nach bekannten Verfahren in einfacher Weise zugänglich.

Die erfindungsgemäßen Verbindungen sind als Photoinitiatoren für photopolymerisierbare Schichten geeignet, die als wesentliche Bestandteile polymerisierbare Verbindungen, Initiatoren und ggf. Bindemittel enthalten.

Die in den erfindungsgemäßen lichtempfindlichen Gemischen verwendeten polymerisierbaren Verbindungen enthalten mindestens eine ethylenische Doppelbindung und können als Monomere, Oligomere, Polymere oder Gemische aus diesen Komponenten vorliegen. Beispiele für geeignete Verbindungen sind ggf. mehrfach ungesättigte Carbonsäuren, deren Salze, Säurederivate, wie Ester oder Amide, Derivate, die sich von der Kohlensäure ableiten, z. B. Urethane, Sulfonyl- oder Phosphinylurethane oder die entsprechenden Harnstoffverbindungen, ungesättigte Ether sowie ungesättigte Derivate, die sich von Epoxiden ableiten lassen.

Beispiele für Carbonsäuren sind Acrylsäure, Methacrylsäure, Itaconsäure, Crotonsäure, Isocrotonsäure und Maleinsäure. Beispiele für Carbonsäuresalze sind die Natrium- und Kaliumsalze der vorstehend genannten Carbonsäuren.

Als Ester von ungesättigten Carbonsäuren mit ggf. mehrwertigen Alkoholen sind Acryl- und Methacrylsäureester, wie Ethylenglykoldi(meth)acrylat, Triethylenglykoldi(meth)acrylat, 1,3-Butandioldi(meth)acrylat, Tetramethylendioldi(meth)acrylat, Propylenglykoldi(meth)acrylat, Trimethylolpropantri(meth)acrylat, Trimethylolethantri(meth)acrylat, 1,4-Cyclohexandioldi(meth)acrylat, Tetraethylenglykoldi(meth)acrylat, Pentaerythritdi(meth)acrylat, Pentaerythrittri(meth)acrylat, Pentaerythrittetra(meth)acrylat, Dipentaerythritdi-(meth)acrylat, Sorbittri(meth)acrylat, Sorbittetra(meth)acrylat, Sorbitpenta(meth)acrylat, Sorbithexa(meth)-acrylat und Polyester(meth)acrylatoligomere, 2,2-Bis-[p-(3-(meth)acryloyloxy-2-hydroxypropoxy)-phenyl]-propan und 2,2-Bis-[(meth)acryloyloxyethoxy-phenyl]-propan; Itaconsäureester, wie Ethylenglykoldiitaconat, Propylenglykoldiitaconat, 1,3-Butandioldiitaconat, 1,4-Butandioldiitaconat, Tetramethylendioldiitaconat, Pentaerythritdiitaconat und Sorbittetraitaconat; Crotonsäureester, wie Ethylenglykoldicrotonat, Tetramethylendioldicrotonat, Pentaerythritdicrotonat und Sorbittetracrotonat; Isocrotonsäureester, wie Ethylenglykoldiisocrotonat, Pentaerythritdiisocrotonat und Sorbittetraisocrotonat; sowie Maleinsäureester, wie Ethylenglykoldimaleinat, Triethylenglykoldimaleinat, Pentaerythritdimaleinat und Sorbittetramaleinat geeignet. Diese Ester können einzeln oder als Gemische eingesetzt werden.

Als Amide von ungesättigten Carbonsäuren mit ggf. mehrwertigen Aminen sind Acryl- und Methacrylamide, z. B. Methylen-bis-(meth)acrylamid, 1,6-Hexamethylen-bis-(meth)acrylamid, Diethylentriamin-tris-(meth)acrylamid und Xylylen-bis-(meth)acrylamid verwendbar.

Weitere bevorzugte polymerisierbare Verbindungen mit mindestens einer ethylenisch ungesättigten Bindung sind Vinylurethanverbindungen mit zwei oder mehreren polymerisierbaren Vinylgruppen im Molekül, die durch Additionsreaktion eines Hydroxyalkyl(meth)acrylats, z. B. Hydroxyethylmethacrylat oder 2-Hydroxy-propylacrylat, mit einem Isocyanat mit mindestens zwei Isocyanatgruppen im Molekül erhalten werden, oder solche, die durch Additionsreaktion aus einem Isocyanatoalkyl(meth)acrylat, z. B. 2-Isocyanatoethylmethacrylat, und einem mehrwertigen, gegebenenfalls Stickstoffatome enthaltenden Alkohol erhalten werden.

Unter den aufgeführten Verbindungen stellen die Acryl-und Methacrylsäureester mehrwertiger Alkohole sowie die Umsetzungsprodukte von Diisocyanaten mit Partialestern mehrwertiger ungesättigter Alkohole und die Umsetzungsprodukte von Hydroxyalkyl(meth)acrylaten mit Polyisocyanaten und Isocyanatoalkyl(meth)-acrylaten mit Polyalkoholen besonders bevorzugte polymerisierbare Komponenten dar. Beispiele für die zuletzt genannten Monomeren sind in den DE-A 20 64 079, 23 61 041 und 28 22 190 beschrieben. Der Mengenanteil der Schicht an Monomeren beträgt im allgemeinen etwa 10 bis 80, vorzugsweise 20 bis 60 Gew.-%, bezogen auf die Menge der nichtflüchtigen Bestandteile.

Beim Einsatz der erfindungsgemäßen Photoinitiatoren in photopolymerisierbaren Gemischen können diese ferner ein Bindemittel enthalten. Das Bindemittel muß mit der ethylenisch ungesättigten polymerisierbaren Verbindung und dem erfindungsgemäßen Photoinitiator verträglich sein. Die lichtempfindliche Schicht muß nach der bildmäßigen Belichtung durch Auswaschen oder Auseinanderziehen (peel apart) verarbeitbar sein. Außerdem sollte das Bindemittel der lichtempfindlichen Schicht ausreichende Zähigkeit, Festigkeit,

Abriebbeständigkeit und Flexibilität verleihen. Gewöhnlich ist das Bindemittel ein lineares organisches Polymeres.

Beispiele für verwendbare Bindemittel sind chloriertes Polyethylen, chloriertes Polypropylen, Poly(meth)acrylsäurealkylester, bei denen die Alkylgruppe z. B. Methyl, Ethyl, n-Butyl, i-Butyl, n-Hexyl oder 2-Ethylhexyl ist, Copolymere der genannten (Meth)acrylsäurealkylester mit mindestens einem Monomeren, wie Acrylnitril, Vinylchlorid, Vinylidenchlorid, Styrol oder Butadien; Polyvinylchlorid, Vinylchlorid/ Acrylnitril-Copolymere, Polyvinylidenchlorid, Vinylidenchlorid/Acrylnitril-Copolymere, Polyvinylacetat, Polyvinylalkohol, Polyacrylnitril, Acrylnitril/Styrol-Copolymere, Acrylnitril/Butadien/Styrol-Copolymere, Polystyrol, Polymethylstyrol, Polyamide (z. B. Nylon-6), Polyurethane, Methylcellulose, Ethylcellulose, Acetylcellulose, Polyvinylformal und Polyvinylbutyral.

Besonders geeignet sind Bindemittel, die in Wasser unlöslich, in organischen Lösungsmitteln löslich und in wäßrig-alkalischen Lösungen löslich oder zumindest quellbar sind.

Besonders erwähnt werden sollen Carboxylgruppen enthaltende Bindemittel, z. B. Copolymerisate aus (Meth)acrylsäure und/oder deren ungesättigten Homologen, wie Crotonsäure, Copolymerisate des Maleinsäureanhydrids oder seiner Halbester, Umsetzungsprodukte hydroxylgruppenhaltiger Polymerer mit Dicarbonsäureanhydriden sowie deren Mischungen.

Weiterhin sind geeignet Umsetzungsprodukte aus Polymeren, die H-acide Gruppen tragen, welche ganz oder teilweise mit aktivierten Isocyanaten umgesetzt wurden, wie beispielsweise Umsetzungsprodukte aus hydroxylgruppenhaltigen Polymeren mit aliphatischen oder aromatischen Sulfonylisocyanaten oder Phosphinsäureisocyanaten.

Darüber hinaus sind geeignet: Hydroxylgruppen enthaltende Polymere, wie beispielsweise Copolymere von Hydroxyalkyl(meth)acrylaten, Copolymere des Allylalkohols, Copolymere des Vinylalkohols, Polyurethane oder Polyester, sowie Epoxyharze, sofern sie eine ausreichende Anzahl von freien OH-Gruppen tragen, oder derart modifiziert sind, daß sie in wäßrig-alkalischen Lösungen löslich sind, oder solche Polymere, die aromatisch gebundene Hydroxylgruppen tragen, wie beispielsweise Kondensationsprodukte von kondensationsfähigen Carbonylverbindungen, insbesondere Formaldehyd, Acetaldehyd oder Aceton, mit Phenolen oder Copolymerisate der Hydroxystyrole. Schließlich lassen sich auch Copolymerisate des (Meth)-acrylsäureamids mit Alkyl(meth)acrylaten verwenden.

Die vorstehend beschriebenen Polymeren sind insbesondere dann geeignet, wenn sie ein Molekulargewicht zwischen 500 und 200.000 oder darüber, bevorzugt 1.000 bis 100.000 aufweisen und entweder Säurezahlen zwischen 10 und 250, bevorzugt von 20 bis 200, oder Hydroxylzahlen zwischen 50 und 750, bevorzugt von 100 bis 500, aufweisen.

Als bevorzugte alkalilösliche Bindemittel seien nachstehend erwähnt:
Copolymerisate der (Meth)acrylsäure mit Alkyl(meth)acrylaten, (Meth)acrylsäurenitril oder dergleichen, Copolymerisate der Crotonsäure mit Alkyl(meth)acrylaten, (Meth)acrylsäurenitril oder dergleichen, Copolymerisate der Vinylessigsäure mit Alkyl(meth)acrylaten, Copolymerisate des Maleinsäureanhydrids mit ggf. substituierten Styrolen, ungesättigten Kohlenwasserstoffen, ungesättigten Ethern oder Estern, Veresterungsprodukte der Copolymerisate des Maleinsäureanhydrids, Veresterungsprodukte von Hydroxylgruppen enthaltenden Polymeren mit Anhydriden von Di- oder Polycarbonsäuren, Copolymerisate der Hydroxyalkyl(meth)acrylate mit Alkyl(meth)acrylaten, (Meth)acrylsäurenitril oder dergleichen, Copolymerisate des Allylalkohols mit ggf. substituierten Styrolen, Copolymerisate des Vinylalkohols mit Alkyl(meth)acrylaten oder anderen polymerisationsfähigen ungesättigten Verbindungen, Polyurethane, sofern sie eine ausreichende Anzahl freier OH-Gruppen aufweisen, Epoxyharze, Polyester, teilverseifte Vinylacetat-Copolymere, Polyvinylacetale mit freien OH-Gruppen, Copolymerisate der Hydroxystyrole mit Alkyl(meth)acrylaten oder dergleichen, Phenol-Formaldehyd-Harze, z. B. Novolake.

Die Menge des Bindemittels in der lichtempfindlichen Schicht beträgt im allgemeinen 20 bis 90, vorzugsweise 40 bis 80 Gew.-%.

Die erfindungsgemäßen Photoinitiatoren werden derartigen Schichten in Mengen zwischen 0,1 und 15,0, bevorzugt von 0,2 bis 5 Gew.-% zugemischt.

Die photopolymerisierbaren Gemische können je nach geplanter Anwendung und je nach den gewünschten Eigenschaften verschiedenartige Stoffe als Zusätze enthalten. Beispiele sind:
Inhibitoren zur Verhinderung der thermischen Polymerisation, Wasserstoffdonatoren, Regler für die spektrale Empfindlichkeit, Farbstoffe, gefärbte und ungefärbte Pigmente, Farbbildner, Indikatoren und Weichmacher.

Geeignete Inhibitoren sind z. B. Hydrochinon, p-Methoxyphenol, Di-t-Butyl-p-kresol, Pyrogallol, t-Butylbrenzkatechin, Benzochinon, Kupfer-I-chlorid, Phenothiazin, Chloranil, Naphthylamin, Naphthol, Nitrobenzol und Dinitrobenzol.

Geeignete Farbstoffe oder Pigmente sind z. B. Methylenblau, Kristallviolett, Rhodamin B, Fuchsin, Aurin, Azofarbstoffe, Anthrachinonfarbstoffe, Titandioxid, Ruß, Eisenoxid, Phthalocyaninpigmente oder Azopigmente, wobei darauf zu achten ist, daß die Absorption des eingesetzten Farbstoffs im Anregungsbereich des Photoinitiators nicht zu stark ist.

Beispiele für Weichmacher sind Phthalsäureester, Glykolester, Phosphorsäureester oder aliphatische Dicarbonsäureester.

Das Gemisch kann außerdem einen Sensibilisator und/oder einen weiteren Photoinitiator enthalten, die so ausgewählt werden, daß die Photopolymerisationsgeschwindigkeit erhöht wird, wenn sie zusammen mit dem Photoinitiator der allgemeinen Formel I verwendet werden. Verwendbare Sensibilisatoren sind Benzoin, Benzoinalkylether, 9-Fluorenon, 2-Brom-9-anthron, 2-Ethyl-9-antrhon, 9,10-Anthrachinon, substituierte Anthrachinone, Xanthon, substituierte Xanthone, Thioxanthon, Benzil, Dibenzalaceton, substituierte Chalkone, Benzophenon oder Benzanthron, Eosin- oder Fluoresceinderivate, Acridine, Pyronine oder ähnliche.

Verwendbare Coinitiatoren sind insbesondere Trichlormethylgruppen enthaltende Photoinitiatoren, deren Absorptionsmaximum deutlich unterhalb der Absorptionsmaxima der erfindungsgemäßen Photoinitiatoren liegt. Besonders geeignet sind z. B. die in der gleichzeitig eingereichten Patentanmeldung P 38 07 380.3 beschriebenen Oxadiazolderivate. Durch diese Maßnahmen läßt sich die spektrale Empfindlichkeit des lichtempfindlichen Gemischs über einen weiten Wellenlängenbereich ausdehnen.

Das photopolymerisierbare Gemisch kann für die verschiedensten Anwendungen Einsatz finden, beispielsweise zur Herstellung von Sicherheitsglas, von Lacken, die durch Licht oder Korpuskularstrahlen, z. B. Elektronenstrahlen, gehärtet werden, auf dem Dentalgebiet und insbesondere als lichtempfindliches Kopiermaterial auf dem Reproduktionsgebiet. Als Anwendungsmöglichkeiten auf diesem Gebiet seien genannt: Kopierschichten für die photomechanische Herstellung von Druckformen für den Hochdruck, den Flexodruck, den Flachdruck, den Tiefdruck, den Siebdruck, von Reliefkopien, z. B. Herstellung von Texten in Blindenschrift, von Einzelkopien, Gerbbildern, Pigmentbildern usw.. Weiter sind die Gemische zur photomechanischen Herstellung von Ätzreservagen, z. B. für die Fertigung von Namensschildern, von gedruckten Schaltungen und für das Formteilätzen, anwendbar.

Die gewerbliche Verwertung des Gemischs für die genannten Anwendungszwecke kann in der Form einer flüssigen Lösung oder Dispersion, z. B. als Photoresistlösung, erfolgen, die vom Verbraucher selbst auf einen individuellen Träger, z. B. zum Formteilätzen, für die Herstellung gedruckter Schaltungen, von Siebdruckschablonen und dgl., aufgebracht wird. Das Gemisch kann auch als feste lichtempfindliche Schicht auf einem geeigneten Träger in Form eines lagerfähig vorbeschichteten lichtempfindlichen Kopiermaterials, z. B. für die Herstellung von Druckformen, vorliegen. Ebenso ist es für die Herstellung von Trockenresist geeignet.

Es ist im allgemeinen günstig, die Gemische während der Lichtpolymerisation dem Einfluß des Luftsauerstoffes weitgehend zu entziehen. Im Fall der Anwendung des Gemischs in Form dünner Kopierschichten ist es empfehlenswert, einen geeigneten, für Sauerstoff wenig durchlässigen Deckfilm aufzubringen. Dieser kann selbsttragend sein und vor der Entwicklung der Kopierschicht abgezogen werden. Für diesen Zweck sind z. B. Polyesterfilme geeignet. Der Deckfilm kann auch aus einem Material bestehen, das sich in der Entwicklerflüssigkeit löst oder mindestens an den nicht gehärteten Stellen bei der Entwicklung entfernen läßt. Hierfür geeignete Materialien sind z. B. Wachse, Polyamide, Polyvinylalkohol, Polyphosphate, Zucker usw..

Als Schichtträger für mit dem erfindungsgemäßen Gemisch hergestellte Kopiermaterialien sind beispielsweise Aluminium, Stahl, Zink, Kupfer und Kunststoff-Folien, z. B. aus Polyethylenterephthalat oder Cellulosacetat, sowie Siebdruckträger, wie Perlongaze, geeignet.

Weiterhin können die erfindungsgemäßen Verbindungen auch in solchen strahlungsempfindlichen Gemischen eingesetzt werden, deren Eigenschaftsänderung durch saure Katalysatoren, die bei der Photolyse des Initiators entstehen, eingeleitet wird. Zu nennen sind etwa die kationische Polymerisation von Systemen, die Vinylether, N-Vinylverbindungen wie N-Vinylcarbazol oder spezielle säurelabile Lactone enthalten, wobei nicht ausgeschlossen wird, daß bei einigen dieser Reaktionen auch radikalische Vorgänge beteiligt sind. Als durch Säuren härtbare Massen sind weiterhin Aminoplaste wie Harnstoff/Formaldehydharze, Melamin/Formaldehydharze und andere N-Methylolverbindungen sowie Phenol/Formaldehydharze zu nennen. Wenn auch die Härtung von Epoxyharzen im allgemeinen durch Lewis-Säuren bzw. solche Säuren erfolgt, deren Anionen eine geringere Nukleophilie als Chlorid besitzen, also als das Anion der bei der Photolyse der neuen Verbindungen entstehenden Halogenwasserstoffsäure, so härten doch Schichten, die aus Epoxyharzen und Novolaken bestehen, bei Belichtung in Gegenwart der erfindungsgemäßen Verbindungen glatt aus.

Eine weitere vorteilhafte Eigenschaft der neuen Verbindungen besteht in ihrer Fähigkeit, in gefärbten Systemen bei der Photolyse Farbumschläge hervorzurufen; aus Farbvorläufern, z. B. Leukoverbindungen,

Farbbildung zu induzieren oder bathochrome Farbverschiebungen und -vertiefungen in Gemischen zu bewirken, die Cyanin-, Merocyanin- oder Styrylfarbbasen enthalten. Auch kann z. B. in den in der DE-A 15 72 080 beschriebenen Gemischen, die Farbbase, N-Vinylcarbazol und einen Halogenkohlenwasserstoff enthalten, die Halogenverbindung Tetrabrommethan durch einen Bruchteil ihrer Menge an erfindungsgemäßer Verbindung ersetzt werden. Farbumschläge sind in der Technik auch z. B. bei der Herstellung von Druckformen erwünscht, um nach der Belichtung bereits vor der Entwicklung das Kopierergebnis beurteilen zu können.

Statt der in den DE-A 23 31 377 und 26 41 100 genannten Säurespender sind vorteilhaft die vorliegenden Verbindungen zu benutzen.

Ein besonders bevorzugtes Anwendungsgebiet für die erfindungsgemäßen Verbindungen sind Gemische, die neben ihnen als wesentliche Komponente eine Verbindung mit mindestens einer durch Säure spaltbaren C-O-C-Gruppierung enthalten. Als durch Säure spaltbare Verbindungen sind in erster Linie zu nennen:

A) solche mit mindestens einer Orthocarbonsäureester-und bzw. oder Carbonsäureamidacetalgruppierung, wobei die Verbindungen auch polymeren Charakter haben und die genannten Gruppierungen als verknüpfende Elemente in der Hauptkette oder als seitenständige Substituenten auftreten können,

B) Polymerverbindungen mit wiederkehrenden Acetal- und/oder Ketalgruppierungen und

C) Polymerverbindungen mit wiederkehrenden Einheiten von aktivierten Estern der Kohlensäure.

Durch Säure spaltbare Verbindungen des Typs A als Komponenten strahlungsempfindlicher Gemische sind in den DE-A 26 10 842 oder 29 28 636 ausführlich beschrieben; Gemische, die Verbindungen des Typs B enthalten, sind Gegenstand der DE-C 27 18 254, und Gemische, die Verbindungen des Typs C enthalten, sind in der EP-A 102 450 beschrieben.

Als durch Säure spaltbare Verbindungen sind z. B. auch die speziellen Aryl-alkyl-acetale und -aminale der DE-C 23 06 248 zu nennen, die durch die Photolyseprodukte der erfindungsgemäßen Verbindungen ebenfalls abgebaut werden.

Solche Gemische, in denen durch Einwirkung von aktinischer Strahlung mittelbar oder unmittelbar Moleküle in kleinere umgewandelt werden, weisen an den bestrahlten Stellen im allgemeinen eine erhöhte Löslichkeit, Klebrigkeit oder Flüchtigkeit auf. Diese Partien können durch geeignete Maßnahmen, beispielsweise Herauslösen mit einer Entwicklungsflüssigkeit, entfernt werden. Bei Kopiermaterialien spricht man in diesen Fällen von positiv arbeitenden Systemen.

Die bei vielen Positiv-Kopiermaterialien bewährten Novolak-Kondensationsharze haben sich als Zusatz auch bei der Anwendung der erfindungsgemäßen Verbindungen in Gemischen mit durch Säure spaltbaren Verbindungen als besonders brauchbar und vorteilhaft erwiesen. Sie fördern die starke Differenzierung zwischen den belichteten und unbelichteten Schichtpartien beim Entwickeln, besonders die höher kondensierten Harze mit substituierten Phenolen als Formaldehyd-Kondensationspartner. Die Art und Menge der Novolak-Harze kann je nach Anwendungszweck verschieden sein; bevorzugt sind Novolak-Anteile am Gesamtfeststoff zwischen 30 und 90, besonders bevorzugt 55 - 85 Gew.-%.

Zusätzlich können noch zahlreiche andere Harze mitverwendet werden, bevorzugt Vinylpolymerisate wie Polyvinylacetate, Polyacrylate, Polyvinylether und Polyvinylpyrrolidone, die selbst durch Comonomere modifiziert sein können. Der günstigste Anteil an diesen Harzen richtet sich nach den anwendungstechnischen Erfordernissen und dem Einfluß auf die Entwicklungsbedingungen und beträgt im allgemeinen nicht mehr als 20 % vom Novolak. In geringen Mengen kann das lichtempfindliche Gemisch für spezielle Erfordernisse wie Flexibilität, Haftung und Glanz etc. außerdem noch Substanzen wie Polyglykole, Cellulose-Derivate wie Ethylcellulose, Netzmittel, Farbstoffe und feinteilige Pigmente sowie bei Bedarf UV-Absorber enthalten. Entwickelt wird vorzugsweise mit in der Technik üblichen wäßrig-alkalischenn Entwicklern, die auch kleine Anteile organischer Lösemittel enthalten können, oder aber mit organischen Lösemitteln.

Die bereits im Zusammenhang mit den photopolymerisierbaren Gemischen aufgeführten Träger kommen ebenfalls für positiv arbeitende Kopiermaterialien in Frage, zusätzlich die in der Mikroelektronik üblichen Silizium-, Siliziumdioxid- oder Galliumarsenidoberflächen.

Die Menge der als Photoinitiator eingesetzten erfindungsgemäßen Verbindungen kann in den positiv arbeitenden Gemischen je nach Substanz und Schicht sehr verschieden sein. Günstigere Ergebnisse werden erhalten zwischen etwa 0,1 und 10 %, bezogen auf den Gesamtfeststoff, bevorzugt sind etwa 0,2 bis 5 %. Für Schichten mit Dicken über 10 µm empfiehlt es sich, relativ wenig Säurespender zu verwenden.

Grundsätzlich ist elektromagnetische Strahlung mit Wellenlängen bis etwa 700 nm zur Belichtung geeignet. Der bevorzugte Wellenlängenbereich erstreckt sich von 300 bis 600 nm. Ihre höchste Empfindlichkeit weisen die erfindungsgemäßen Gemische im Bereich zwischen 350 und 550 nm auf.

Die Vielfalt der erfindungsgemäßen Verbindungen, deren Absorptionsmaxima häufig im sichtbaren Teil des Spektrums zu finden sind und deren spektrale Empfindlichkeit häufig über 600 nm hinausreicht, gestattet es, den Photoinitiator optimal auf die verwendete Lichtquelle abzustimmen. Als Lichtquellen sind beispielsweise zu nennen: Röhrenlampen, Xenonimpulslampen, metallhalogeniddotierte Quecksilberdampf-Hochdrucklampen und Kohlebogenlampen.

Darüber hinaus ist bei den erfindungsgemäßen lichtempfindlichen Gemischen das Belichten in üblichen Projektions- und Vergrößerungs-Geräten unter dem Licht von Metallfadenlampen und Kontaktbelichtung mit gewöhnlichen Glühbirnen mit Vorteil möglich. Die Belichtung kann auch mit kohärentem Licht eines Lasers erfolgen. Geeignet für die Zwecke vorliegender Erfindung sind beispielsweise Argon-Ionen-Laser, wobei eine praxisgerechte Ausnutzung der Emission bei 488 bzw. 514 nm gewährleistet ist, Krypton-Ionen-Laser, Farbstoff-Laser, Helium-Cadmium- oder Helium-Neon-Laser, wobei die Linien zwischen 400 und 650 nm mit besonderem Vorteil genutzt werden können. Der Laserstrahl wird im allgemeinen mittels einer vorgegebenen programmierten Strich- und/oder Raster-Bewegung gesteuert.

Das Bestrahlen mit Elektronenstrahlen ist eine weitere Differenzierungsmöglichkeit. Elektronenstrahlen können Gemische, die eine der erfindungsgemäßen Verbindungen und eine durch Säure spaltbare Verbindung enthalten, wie auch viele andere organische Materialien durch-greifend zersetzen und vernetzen, so daß ein negatives Bild entsteht, wenn die unbestrahlten Teile durch Lösemittel oder Belichten ohne Vorlage und Entwickeln entfernt werden.

Bei geringerer Intensität und/oder höherer Schreibgeschwindigkeit des Elektronenstrahls bewirkt dagegen der Elektronenstrahl eine Differenzierung in Richtung höherer Löslichkeit, d. h. die bestrahlten Schichtpartien können vom Entwickler entfernt werden. Die günstigsten Bedingungen können durch Vorversuche leicht ermittelt werden.

Bevorzugte Anwendung finden die die erfindungsgemäßen Verbindungen enthaltenden strahlungsempfindlichen Gemische bei der Herstellung von Druckformen, d. h. insbesondere Offset-, Hoch-, Flexo-, autotypischen Tiefdruck- und Siebdruckformen, in Photoresistlösungen und in Trockenresists.

Aufgrund der Eigenschaften der erfindungsgemäßen Verbindungen erhält man dabei lichtempfindliche Aufzeichnungsmaterialien, die gegenüber dem Stand der Technik zahlreiche Vorteile haben. Unter diesen ist besonders die Belichtbarkeit mit im sichtbaren Bereich strahlenden Lichtquellen zu nennen, wobei Empfindlichkeiten des lichtempfindlichen Aufzeichnungsmaterials erhalten werden, welche denen der bekannten Gemische, die nur im nahen UV-Bereich empfindlich sind, mindestens ebenbürtig sind. Dadurch ist die praxisgerechte, rasche Belichtung mit wenig energiereichen, im sichtbaren Bereich emittierenden Lasern gewährleistet. Die häufig bei längerwellig ansprechbaren lichtempfindlichen Gemischen beobachtete hohe Empfindlichkeit gegenüber Luftsauerstoff wird bei Einsatz der erfindungsgemäßen Photoinitiatoren nur in untergeordnetem Maß beobachtet. Ganz besonders vorteilhaft ist jedoch der Umstand, daß die hohe chemische und thermische Stabilität der erfindungsgemäßen Photoinitiatoren eine ungewöhnlich hohe Lagerstabilität der lichtempfindlichen Schicht bewirkt, die eine Vorratshaltung und längere Lagerung der Lösungen bzw. lichtempfindlichen Schichten erlaubt. Schließlich ist als ein weiterer wichtiger Vorteil noch zu vermerken, daß die lichtempfindlichen Gemische bei Verwendung der erfindungsgemäßen Photoinitiatoren bzw. Säurespender praktisch keine katalytisch ausgelösten Dunkelreaktionen auf kritischen Oberflächen, wie z. B. Kupferoberflächen, erleiden, was an sich schon eine Verbesserung gegenüber dem Stand der Technik darstellt.

Obwohl die lichtempfindlichen Gemische gegenüber sichtbarem Licht stark empfindlich sind und daher unter geeigneten Bedingungen, im Extremfall unter Rotlicht, gehandhabt werden müssen, sind die erfindungsgemäßen Verbindungen in kristallinem Zustand ungewöhnlich licht- und thermostabil. Sie lassen sich daher ohne größeren Aufwand unter praktisch normalen Produktionsbedingungen herstellen und handhaben und sind in diesem Zustand lange stabil, so daß eine praxisgerechte Bevorratung mit diesen Verbindungen möglich ist.

Die folgenden Beispiele dienen zur näheren Erläuterung der Erfindung; es erfolgt zunächst die Beschreibung der Herstellung von verschiedenen erfindungsgemäßen Verbindungen, woran sich die Anwendung von einigen dieser Verbindungen in strahlungsempfindlichen Gemischen anschließt.

In den Beispielen stehen Gew.-Teile (Gt) und Vol.-Teile (Vt) im Verhältnis von g zu ml. Prozent- und Mengenangaben sind, wenn nichts anderes angegeben ist, in Gewichtseinheiten zu verstehen.

Herstellungsbeispiel 1

a) 2,3-Dimethyl-benzthiazolium-p-toluolsulfonat

74,6 Gt 2-Methylbenzthiazol und 93,1 Gt Toluolsulfonsäuremethylester werden miteinander vermischt und unter Rühren erwärmt. Ab etwa 120 °C beginnt eine exotherme Reaktion, bei der sich das Gemisch selbständig auf 180 °C erwärmt. Die Lösung wird 10 Minuten bei dieser Temperatur belassen und dann vorsichtig in 1000 Gt Aceton gegossen. Die erhaltene Suspension wird eine Stunde nachgerührt. Der Niederschlag wird abgesaugt und mit Aceton gewaschen. Das erhaltene Pulver wird im Vakuum über Phosphorpentoxid getrocknet. Ausbeute: 147,2 Gt = 87,8 % d. Th.

b) 2-[4-(4,6-Bis-trichlormethyl-s-triazin-2-yl)benzoylmethylen]-3-methyl-benzthiazolin (Verbindung 1)

In 150 Gt Toluol werden nacheinander 13,4 Gt 2,3-Dimethyl-benzthiazolium-p-toluolsulfonat und 22,7 Gt 4-(4,6-Bis-trichlormethyl-s-triazin-2-yl)benzoylchlorid suspendiert bzw. gelöst. Die Mischung wird auf 15 °C gekühlt, und bei dieser Temperatur werden unter Feuchtigkeitsausschluß 13,1 Gt Triethylamin zugetropft. Die sich nach dunkelbraun verfärbende Mischung wird 3 Stunden bei Raumtemperatur nachgerührt. Der gebildete Niederschlag wird abgesaugt und mit kaltem Toluol nachgewaschen. Der Rückstand wird in 200 Gt Methanol digeriert und etwa 60 Minuten gerührt, erneut filtriert und getrocknet.

Das Produkt wird aus 2-Methoxy-ethanol umkristallisiert. Orangefarbene Kristalle, Ausbeute: 22 Gt = 96,9 % d. Th., Smp. 243 °C (Zersetzung).

| $C_{21}H_{12}Cl_6N_4OS$ (581,1) | | | | | |
|---|---|---|---|---|---|
| ber:<br>gef: | C 43,40<br>C 43,4 | H 2,08<br>H 2,0 | N 9,64<br>N 9,6 | S 5,52<br>S 5,7 | Cl 36,60<br>Cl 35,9 |

UV ($CH_2Cl_2$): $\lambda_{max}$ = 426 nm.

Herstellungsbeispiel 2:

a) 3-Ethyl-2-methyl-benzthiazolium-p-toluolsulfonat

Unter Rühren werden 300 Gt 2-Methylbenzthiazol und 440 Gt p-Toluolsulfonsäureethylester auf 150 °C erhitzt, wobei durch exotherme Reaktion die Temperatur auf ca. 200 °C steigt. Nach 10 Minuten wird in 2000 Gt Aceton gegossen, das ausgefallene Produkt abgesaugt, mit Aceton gewaschen und über Phosphorpentoxid getrocknet. Ausbeute: 730 Gt = 98 % d. Th.

b) 2-(Bis-[4-(4,6-Bis-trichlormethyl-s-triazin-2-yl)benzoyl]-methylen)-3-ethyl-benzthiazolin (Verbindung 38)

Zu 7 Gt 2-Methyl-3-ethyl-benzthiazolium-p-toluolsulfonat in 30 Gt trockenem Pyridin werden bei 8 °C 22,7 Gt 4-(4,6-Bis-trichlormethyl-s-triazin-2-yl)benzoylchlorid zugetropft. Die Mischung wird unter Rühren zum Rückfluß erhitzt und nach 150 Minuten abkühlen gelassen. Das Pyridin wird im Vakuum abdestilliert und der Rückstand mit 100 Gt Methanol versetzt. Die Mischung wird 1 Stunde digeriert, der Rückstand abfiltriert und mit Methanol gewaschen. Nach Trocknung wird aus Acetonitril umkristallisiert. Ausbeute: 11,3 Gt = 56 % d. Th. Gelbe Kristalle, Smp. 146 - 147 °C.

| $C_{34}H_{17}Cl_{12}N_7O_2S$ (1013,0) | | | | | |
|---|---|---|---|---|---|
| ber:<br>gef: | C 40,31<br>C 40,6 | H 1,69<br>H 1,7 | N 9,68<br>N 9,8 | S 3,20<br>S 3,0 | Cl 42,00<br>Cl 42,5 |

UV ($CH_2Cl_2$): $\lambda_{max}$ = 385 nm.

Herstellungsbeispiel 3:

2-[4-(4,6-Bis-trichlormethyl-s-triazin-2-yl)-benzoylmethylen]-1,3,3-trimethyl-indolin (Verbindung 29)

Zu 5,2 Gt 1,3,3-Trimethyl-2-methylen-indolin und 10,6 Gt Triethylamin in 140 Gt wasserfreiem Toluol werden unter Rühren bei 10 °C 15,4 Gt 4-(4,6-Bis-trichlormethyl-s-triazin-2-yl)-benzoylchlorid zugetropft. Die Mischung wird 3 Stunden bei Raumtemperatur nachgerührt. Das Gemisch wird mit 100 Gt Wasser und 200 Gt Ether versetzt und ausgeschüttelt. Die getrocknete organische Phase wird eingeengt. Der Rückstand wird aus 2-Methoxyethanol umkristallisiert. Rotorange Kristalle. Ausbeute 10,2 Gt = 57 % d. Th., Smp. 215 °C (Zersetzung).

| $C_{24}H_{18}Cl_6N_4O$ (591,2) | | | | |
|---|---|---|---|---|
| ber: | C 48,76 | H 3,07 | N 9,48 | Cl 36,00 |
| gef: | C 48,5 | H 3,0 | N 9,4 | Cl 36,6 |

UV ($CH_2Cl_2$): $\lambda_{max}$ = 422 nm.

Herstellungsbeispiel 4

2-[3-(4,6-Bis-trichlormethyl-s-triazin-2-yl)-benzoylmethylen]-3-ethyl-benzthiazolin (Verbindung 25)

In 50 Gt Toluol werden nacheinander 5,0 Gt 3-Ethyl-2-methyl-benzothiazolium-p-toluolsulfonat und 7,4 Gt 3-(4,6-Bis-trichlormethyl-s-triazin-2-yl)-benzoylchlorid suspendiert bzw. gelöst. Die Mischung wird auf 15 °C gekühlt, und bei dieser Temperatur werden unter Feuchtigkeitsausschluß 6,0 Gt Triethylamin zugetropft. Die sich nach dunkelbraun verfärbende Mischung wird 3 Stunden bei Raumtemperatur nachgerührt. Der gebildete Niederschlag wird abgesaugt und mit kaltem Toluol nachgewaschen. Der Rückstand wird in 200 Gt Methanol digeriert und etwa 60 Minuten gerührt, erneut filtriert und getrocknet. Das Produkt wird aus 2-Methoxy-ethanol umkristallisiert. Orangefarbene Kristalle, Ausbeute: 6,2 Gt = 72,8 % d. Th., Smp. 191,5 - 193,0 °C (Zersetzung).

| $C_{22}H_{14}Cl_6N_4OS$ (595,1) | | | | | |
|---|---|---|---|---|---|
| ber: | C 44,40 | H 2,37 | N 9,41 | S 5,39 | Cl 35,74 |
| gef: | C 44,4 | H 2,3 | N 9,3 | S 5,6 | Cl 35,4 |

UV ($CH_2Cl_2$): $\lambda_{max}$ = 385 nm.

Herstellungsbeispiel 5:

a) 1,2-Dimethyl-chinolinium-p-toluolsulfonat

71,5 Gt 2-Methyl-chinolin und 102,3 Gt p-Toluolsulfonsäuremethylester werden ca. 10 Minuten auf 100 °C erwärmt. Die einsetzende exotherme Reaktion erhitzt das Gemisch auf 180 °C. Nach weiteren 10 Minuten wird auf Aceton gegossen, abgesaugt, mit Aceton gewaschen und getrocknet. Ausbeute: 150 Gt = 92 % d. Th.

b) 2-[4-(4,6-Bis-trichlormethyl-s-triazin-2-yl)-benzoylmethylen]-1-methyl-1,2-dihydrochinolin (Verbindung 39)

14,1 Gt 1,2-Dimethyl-chinolinium-p-toluolsulfonat und 22,2 Gt 4-(4,6-Bis-trichlormethyl-s-triazin-2-yl)-benzoylchlorid werden in 150 ml getrocknetem Toluol vorgelegt und bei 15 °C tropfenweise mit 17,4 Gt Triethylamin versetzt. Dann wird auf Raumtemperatur erwärmen gelassen. Nach 1 Stunde wird die Mischung auf 600 Gt Wasser gegossen, mit 600 Gt Methylenchlorid versetzt und ausgeschüttelt. Der getrockneten organischen Phase werden 100 Gt Kieselgel zugeführt, und die Mischung wird 30 Minuten gerührt. Das Kieselgel wird abfiltriert, und die Lösungsmittel werden unter Vakuum abdestilliert. Der Rückstand wird aus 2-Methoxy-ethanol umkristallisiert.
Ausbeute: 16,3 Gt = 66,1 % d. Th. Intensiv rote Kristalle, Smp. 240 °C (Zersetzung)

| $C_{23}H_{14}Cl_6N_4O$ (575,1) | | | | |
|---|---|---|---|---|
| ber: | C 48,03 | H 2,45 | N 9,74 | Cl 36,99 |
| gef: | C 47,9 | H 2,3 | N 9,6 | Cl 36,7 |

UV ($CH_2Cl_2$): $\lambda_{max}$ = 460 nm.

Herstellungsbeispiel 6

a) 4-(Biphenyl-4-yl)-3-ethyl-2-methyl-thiazolium-p-toluolsulfonat

9,8 Gt 4-(Biphenyl-4-yl)-2-methyl-thiazol und 8,6 Gt p-Toluolsulfonsäureethylester werden 3 Stunden bei 180 °C gerührt. Die heiße Mischung wird auf Aceton gegossen und wie im Herstellungsbeispiel 5 (a) beschrieben aufgearbeitet.

b) 2-[4-(4,6-Bis-trichlormethyl-s-triazin-2-yl)-benzoylmethylen]-4-(biphenyl-4-yl)-3-ethyl-thiazolin (Verbindung 33)

Entsprechend Herstellungsbeispiel 1 werden 5 Gt der unter (a) beschriebenen Verbindung, 5,9 Gt 4-(4,6-Bis-trichlormethyl-s-triazin-2-yl)-benzoylchlorid und 3,5 Gt Triethylamin in 50 Gt Toluol miteinander umgesetzt und aufgearbeitet. Die Umkristallisation erfolgt aus Acetonitril. Ausbeute: 5 Gt = 63 % d. Th. Rote Kristalle, Smp. 205 - 206 °C (Zersetzung).

| $C_{30}H_{20}Cl_6N_4OS$ (697,3) | | | | | |
|---|---|---|---|---|---|
| ber: | C 51,68 | H 2,89 | N 8,03 | S 4,60 | Cl 30,51 |
| gef: | C 51,7 | H 3,1 | N 7,8 | S 4,6 | Cl 29,3 |

UV ($CH_2Cl_2$): $\lambda_{max}$ = 430 nm.

Die in der nachstehenden Tabelle I aufgeführten Verbindungen wurden nach den in den Herstellungsbeispielen 1 bis 7 ausgeführten Herstellungsmethoden synthetisiert.

Die Verbindungen Nr. 1 bis Nr. 24 wurden entsprechend Herstellungsbeispiel 1, die Verbindungen 25 bis 28 entsprechend Herstellungsbeispiel 4, die Verbindungen 29 bis 31 entsprechend Herstellungsbeispiel 3, die Verbindungen 32 bis 36 entsprechend Herstellungsbeispiel 5, die Verbindungen 37 und 38 entsprechend Herstellungsbeispiel 2 und die Verbindung 39 entsprechend Herstellungsbeispiel 6 hergestellt.

Tabelle I

Verbindungen der allgemeinen Formel I

| Verbindung | Q | M | $R^1$ | $R^2$ | L | n |
|---|---|---|---|---|---|---|
| 1 | S | 1,2-Phenylen | $CH_3$ | H | H | O |
| 2 | " | " | $C_2H_5$ | " | " | " |
| 3 | " | " | $C_3H_7$ | " | " | " |
| 4 | " | " | $C_4H_9$ | " | " | " |
| 5 | " | " | $C_5H_{11}$ | " | " | " |
| 6 | " | " | $C_6H_{13}$ | " | " | " |
| 7 | " | " | $CH(CH_3)_2$ | " | " | " |
| 8 | " | " | $C_6H_{11}$ | " | " | " |
| 9 | " | " | $CH_2C_6H_5$ | " | " | " |
| 10 | " | " | $C_2H_4OCH_3$ | " | " | " |
| 11 | " | " | $C_2H_4OC_2H_5$ | " | " | " |
| 12 | " | " | $C_2H_4OC_6H_5$ | " | " | " |
| 13 | O | " | $C_2H_5$ | " | " | " |
| 14 | Se | " | $CH_3$ | " | " | " |
| 15 | " | " | $C_2H_5$ | " | " | " |
| 16 | " | " | $C_5H_{11}$ | " | " | " |
| 17 | S | 1,2-Naphthylen | $CH_3$ | " | " | " |
| 18 | " | " | $C_5H_{11}$ | " | " | " |
| 19 | O | 2,3-Naphthylen | $CH_3$ | " | " | " |
| 20 | " | " | $C_5H_{11}$ | " | " | " |
| 21 | S | 3,4-Tolylen | $C_2H_5$ | " | " | " |
| 22 | " | 4-Methoxy-1,2-phenylen | $CH_3$ | " | " | " |
| 23 | Se | 3,4-Tolylen | $CH_3$ | " | " | " |

Fortsetzung der Tabelle I

Verbindungen der allgemeinen Formel I

| Verbindung | Q | M | $R^1$ | $R^2$ | L | n |
|---|---|---|---|---|---|---|
| 24 | Se | 5-Methoxy-1,2-phenylen | $CH_3$ | H | H | 0 |
| 25 | S | 1,2-Phenylen | $C_2H_5$ | X | " | " |
| 26 | " | 1,2-Naphthylen | $C_5H_{11}$ | " | " | " |
| 27 | Se | 1,2-Phenylen | $C_2H_5$ | " | " | " |
| 28 | S | " | " | " | " | 1 |
| 29 | $C(CH_3)_2$ | " | $CH_3$ | H | " | 0 |
| 30 | " | 4-Chlor-1,2-phenylen | " | " | " | " |
| 31 | " | 4-Nitro-1,2-phenylen | " | " | " | " |
| 32 | S | 1-Phenyl-ethen-1,2-ylen | $C_2H_5$ | " | " | " |
| 33 | " | 1-Biphenyl-4-yl-ethen-1,2-ylen | " | " | " | " |
| 34 | " | 1-Tol-4-yl-ethen-1,2-ylen | " | " | " | " |
| 35 | " | 1-(4-methoxy-phenyl)-ethen-1,2-ylen | " | " | " | " |
| 36 | " | 1,2-Diphenyl-ethen-1,2-ylen | " | " | " | " |
| 37 | " | 1,2-Phenylen | $CH_3$ | " | Y | " |
| 38 | " | " | $C_2H_5$ | " | " | " |
| 39 | -CH=CH- | " | $CH_3$ | " | H | " |

X = 4,6-Bis-trichlormethyl-s-triazin-2-yl

Y = $-CO-(CH=CH)_n-$ 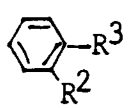

Die Stellungsbezeichnung bei M ist so gewählt, daß der Rest mit der niedrigeren Bezifferung am Stickstoffatom steht.

Tabelle II

| Die Verbindungen 1 bis 39 - soweit sie nicht schon beschrieben wurden - weisen Absorptionsmaxima bei folgenden Wellenlängen auf (Lösungsmittel $CH_2Cl_2$): | | | |
|---|---|---|---|
| Nr. | $\lambda_{max}$ (nm) | Nr. | $\lambda_{max}$ (nm) |
| 2 | 422 | 19 | 390 |
| 3 | 425 | 20 | 401 |
| 4 | 428 | 21 | 429 |
| 5 | 428 | 22 | 431 |
| 6 | 425 | 23 | 432 |
| 7 | 426 | 24 | 435 |
| 8 | 424 | 26 | 388 |
| 9 | 429 | 27 | 390 |
| 10 | 428 | 28 | 416 |
| 11 | 428 | 30 | 420 |
| 12 | 426 | 31 | 427 |
| 13 | 400 | 32 | 428 |
| 14 | 429 | 34 | 432 |
| 15 | 430 | 35 | 440 |
| 16 | 430 | 36 | 430 |
| 17 | 448 | 37 | 384 |
| 18 | 450 | | |

Die Anwendungsbeispiele 1 bis 10 sollen belegen, daß die erfindungsgemäßen Photoinitiatoren bei Verwendung in lichtempfindlichen Mischungen, die mit heute üblicherweise angewendeten, im nahen UV-Bereich emittierenden Lichtquellen belichtet werden, den bekannten Photoinitiatoren gleichwertig sind.

Anwendungsbeispiel 1

Eine elektrochemisch aufgerauhte und anodisierte Aluminiumplatte mit einer Oxidschicht von 2,5 $g/m^2$ wurde mit einer wäßrigen Lösung von Polyvinylphosphonsäure vorbehandelt. Das Trägermaterial wurde mit einer Lösung nachstehender Zusammensetzung überzogen:

91,2 Gt einer 31 %igen Lösung eines Terpolymerisats aus Styrol, n-Hexylmethacrylat und Methacrylsäure (10:60:30) mit der Säurezahl 190 in Butanon,

54,9 Gt einer 51,5 %igen Lösung des Umsetzungsprodukts aus 1 mol Hexamethylendiisocyanat und 2 mol Hydroxyethylmethacrylat,

2,72 Gt der Verbindung Nr. 1 und

660 Gt 2-Methoxy-ethanol

Es wurde durch Aufschleudern und 2 Minuten Trocknen bei 100 °C ein Trockenschichtgewicht von 3,0 $g/m^2$ erhalten. Die lichtempfindliche Schicht wurde mit einer Polyvinylalkoholdeckschicht überzogen.

Die erhaltene Druckplatte wurde mittels einer 5 kW-Metallhalogenidlampe im Abstand von 110 cm unter einem 13-stufigen Belichtungskeil mit zusätzlichen Strich-und Rasterelementen 5 Sekunden belichtet. Nach dieser Belichtung wurde die Platte eine Minute auf 100 °C erwärmt.

Anschließend wurde mit einem Entwickler nachstehender Zusammensetzung entwickelt:

60 Gt Natriummetasilikat x 9 $H_2O$,

1,06 Gt Strontiumchlorid x 6 $H_2O$,

0,6 Gt nichtionogenes Netzmittel und

2000 Gt vollentsalztes Wasser.

Die Platte war bis zur Stufe 5 vernetzt. Die Wiedergabe der feinen Strich- und Rasterelemente war in zufriedenstellender Weise gegeben. Die Platte nahm beim Einspannen in eine Bogenoffsetmaschine die angebotene Farbe willig an und ergab eine Druckauflage von mehr als 100.000.

Anwendungsbeispiel 2

Es wurde eine Beschichtungslösung nachstehender Zusammensetzung bereitet und auf eine wie im Anwendungsbeispiel 1 beschrieben vorbehandelte Aluminiumplatte zu einem Trockenschichtgewicht von 2,8

$g/m^2$ aufgetragen:
102,6 Gt eines Copolymeren aus Methylmethacrylat und Methacrylsäure (82:18) mit der Säurezahl 118, zugesetzt als 34,4 %ige Lösung in Butanon,
36 Gt Trimethylolethantriacrylat,
0,7 Gt eines blauen Azofarbstoffs, erhalten durch Kuppeln von 2,4-Dinitro-6-chlor-benzoldiazoniumsalz mit 2-Methoxy-5-acetylamino-N-cyanoethyl-N-hydroxyethyl-anilin und
1,56 Gt der Verbindung 39 in
462 Gt 2-Methoxy-ethanol

Die lichtempfindliche Schicht wurde mit einer Deckschicht aus Polyvinylalkohol überzogen, 30 Sekunden wie im Anwendungsbeispiel 1 beschrieben belichtet und ohne zusätzliches Erwärmen mit dem dort angegebenen Entwickler entwickelt.

Man erhielt eine Platte mit hoher Auflösung, die beim Einspannen in eine Bogenoffsetmaschine knapp 200.000 Drucke lieferte.

## Anwendungsbeispiel 3

Wie im Anwendungsbeispiel 1 beschrieben, wurde eine lichtempfindliche Lösung nachstehender Zusammensetzung auf ein Aluminiumblech zu einem Trockenschichtgewicht von 3,0 $g/m^2$ aufgebracht und mit einer Deckschicht überzogen:
32,83 Gt eines Umsetzungsprodukts aus einem Polyvinylbutyral mit 71 Gew.-% Vinylbutyral-, 2 Gew.-% Vinylacetat- und 27 Gew.-% Vinylalkoholeinheiten und Propenylsulfonylisocyanat mit der Säurezahl 145, zugesetzt als 12 %ige Lösung in Tetrahydrofuran,
0,03 Gt des im Anwendungsbeispiel 2 angegebenen blauen Azofarbstoffs,
3,94 Gt des im Anwendungsbeispiel 1 beschriebenen Monomeren und
0,37 Gt der Verbindung Nr. 29 in
87,42 Gt 2-Methoxy-ethanol

Belichtung und Entwicklung erfolgten wie im Anwendungsbeispiel 1 angegeben. Auch hier wurde bei einer Belichtungszeit von 8 Sekunden eine vollvernetzte Stufe 5 beobachtet; alle Raster- und Strichelemente waren in zufriedenstellender Weise wiedergegeben. Beim Druck wurden 145.000 Bögen erzielt.

## Anwendungsbeispiel 4

Eine Lösung von
66 Gt des im Anwendungsbeispiel 1 beschriebenen Terpolymerisats,
42 Gt Polypropylenglykol-420-dimethacrylat,
0,2 Gt des im Anwendungsbeispiel 2 angegebenen Farbstoffs,
2,5 Gt der Verbindung Nr. 15 in
240 Gt Butanon und
30 Gt 2-Methoxy-ethanol
wurde auf eine mit 35 µm dicker Kupferfolie kaschierte Phenoplast-Schichtstoffplatte so aufgeschleudert, daß nach dem Trocknen bei 100 °C eine Schichtdicke von 45 µm erhalten wurde. Die Platte wurde mittels einer 5 kW-Metallhalogenidlampe im Abstand von 110 cm zwischen Lampe und Vakuumrahmen 40 Sekunden belichtet. Als Vorlage diente sowohl ein 13-stufiger Belichtungskeil mit Dichteinkrementen von 0,15 als auch eine Strichvorlage mit Linienbreiten und Abständen bis herab zu 80 µm.

Nach der Belichtung wurde die Schicht mit 0,8 %iger Natriumcarbonatlösung in einem Sprühentwicklungsgerät 100 Sekunden lang entwickelt. Es wurden 5 vollvernetzte Keilstufen erhalten.

Die Platte wurde dann 30 Sekunden mit Leitungswasser gespült, 30 Sekunden mit einer 15 %igen Ammoniumperoxydisulfat-Lösung geätzt, erneut mit Wasser gespült, 30 Sekunden in 10 %ige Schwefelsäure getaucht und sodann nacheinander in den folgenden Elektrolytbädern galvanisiert:

1) 50 Minuten in einem Kupferelektrolytbad der Firma Schlötter, Geislingen/Steige
Typ "Glanzkupferbad PC".
Stromdichte: 2,5 $A/dm^2$
Metallaufbau: ca. 25 µm
Temperatur: Raumtemperatur

2) 15 Minuten in einem Bleizinnbad LA der Firma Schlötter, Geislingen/Steige
Stromdichte: 2 $A/dm^2$
Metallaufbau: 15 µm
Temperatur: Raumtemperatur

Die Platte zeigte keine Unterwanderungen oder Beschädigungen. Der Überhang bzw. die Neigung einer Flanke der Resistschicht betrug weniger als 10 µm bei einer Resistbahnbreite von 140 µm.

Die Resistschablone konnte dann in 5 %iger KOH-Lösung bei 50 °C entfernt und das freigelegte Kupfer in den üblichen Ätzmedien weggeätzt werden.

Anwendungsbeispiel 5

Auf eine mechanisch aufgerauhte Aluminiumplatte wurde eine Lösung aus
75 Gt eines Kresol-Formaldehyd-Novolaks mit einem Schmelzbereich von 105 - 120 °C,
23,8 Gt eines Polyacetals aus Triethylenglykol und 2-Butyraldehyd,
0,02 Gt Kristallviolettbase und
0,6 Gt der Verbindung Nr. 25 in
24 Gt 2-Methoxy-ethanol und
275 Gt Butanon
aufgeschleudert und getrocknet. Es wurde durch eine Vorlage belichtet, die einen Stufenkeil und feine Strich- und Rasterelemente aufwies. Die Entwicklung erfolgte mit einer Lösung aus
5,5 Gt Natriummetasilikat x 9 $H_2O$,
3,4 Gt Trinatriumphosphat x 12 $H_2O$ und
0,4 Gt Natriumdihydrogenphosphat in
90,7 Gt entsalztem Wasser.

Bei einer Belichtungszeit von 50 Sekunden und Entwickeln nach 10 Minuten Wartezeit wurden 5 vollentwickelte Keilstufen erhalten. Die Wiedergabe der Testelemente erfolgte bis herab in den 10 µm-Bereich.

Anwendungsbeispiel 6

Es wurde eine Positiv-Trockenresistlösung nachstehender Zusammensetzung hergestellt:
21,2 Gt des im Anwendungsbeispiel 5 beschriebenen Novolaks,
10 Gt des Bis-(5-ethyl-5-butyl-1,3-dioxan-2-yl)ethers von 2-Ethyl-2-butyl-1,3-propandiol,
0,05 Gt Kristallviolettbase,
3,8 Gt Polyethylacrylat, niedrigviskos und
0,25 Gt der Verbindung Nr. 2 in
65 Gt Butanon.

Damit wurde eine mit wäßriger Trichloressigsäure/Polyvinylalkohol-Lösung vorbehandelte biaxial verstreckte und thermofixierte 25 µm dicke Polyesterfolie beschichtet. Das Trockenschichtgewicht betrug 45 g/m². Diese Schicht wurde beidseitig auf ein Kupferblech laminiert, und nach dem Abkühlen, Abziehen der Trägerfolie und Nachtrocknen im Trockenschrank bei 80° C wurde das Blech mit einem deckungsgleichen Vorlagenpaar in Form einer Tasche beidseitig belichtet. Durch Sprühentwicklung unter Verwendung der im Anwendungsbeispiel 5 beschriebenen Entwicklerlösung wurden die belichteten Schichtpartien entwickelt. Die Platte wurde beidseitig mit handelsüblicher Ferrichloridlösung geätzt, bis sie sauber durchgeätzt war. Die Resistschablonen wurden mit einer 4 %igen KOH-Lösung entfernt, und man erhielt ein Formätzteil, das die Vorlage einwandfrei wiedergab.

Anwendungsbeispiel 7 und Vergleichsbeispiele 8 bis 10

Die im Anwendungsbeispiel 1 angegebene Beschichtungslösung wurde unter Ersatz der Verbindung Nr. 1 durch die äquimolare Menge jeweils eines der nachstehend angegebenen Photoinitiatoren auf den dort beschriebenen Träger aufgebracht.

| | |
|---|---|
| Anwendungsbeispiel 7: | Verbindung Nr. 2, |
| Vergleichsbeispiel 8: | 2-(p-Trichlormethyl-benzoylmethylen)-3-ethyl-benzthiazolin (Verbindung Nr. XII der EP-A 135 348) |
| Vergleichsbeispiel 9: | 2-(4-Methoxy-naphth-1-yl)-4,6-bis-trichlor-methyl-s-triazin (Verbindung Nr. 3 der DE-C 27 18 259) |
| Vergleichsbeispiel 10: | 2-(4-Methoxy-styryl)-5-trichlormethyl-1,3,4-oxadiazol (Verbindung Nr. 7 der DE-C 28 51 471) |

Die Belichtung erfolgte mit der in Beispiel 1 beschriebenen, in der Praxis sehr häufig eingesetzten Metallhalogenid-Lampe, wobei die Vorlage durch einen Graufilm abgedeckt wurde, der die tatsächliche Belichtungszeit um den Faktor 10 erhöht.

Alle Muster wurden 40 Sekunden lang belichtet und wie im Anwendungsbeispiel 1 angegeben weiterverarbeitet. Man erhielt folgende Ergebnisse:

Anwendungsbeispiel 7: 5 vollvernetzte Keilstufen,
Vergleichsbeispiel 8: 4 vollvernetzte Keilstufen, Stufe 5 teilvernetzt,
Vergleichsbeispiel 9: 5 vollvernetzte Keilstufen,
Vergleichsbeispiel 10: 2 vollvernetzte Keilstufen, Stufe 3 teilvernetzt.

Daraus ergibt sich, daß der erfindungsgemäße Photoinitiator den bekannten Photoinitiatoren bei Belichtung mit handelsüblichen, im nahen UV-Bereich emittierenden Lichtquellen mindestens gleichwertig oder sogar überlegen ist.

Anhand der nachfolgenden Anwendungsbeispiele 11 bis 20 soll die Überlegenheit der erfindungsgemäßen Photoinitiatoren bezüglich ihrer Empfindlichkeit im sichtbaren Bereich belegt werden.

## Anwendungsbeispiel 11 und Vergleichsbeispiele 12 bis 15

Die im Anwendungsbeispiel 1 angegebene Mischung wurde unter Ersatz der Verbindung 1 durch eine äquimolare Menge eines der nachstehend beschriebenen Photoinitiatoren auf den dort beschriebenen Träger aufgebracht.

Anwendungsbeispiel 11: Verbindung Nr. 2,
Vergleichsbeispiel 12: 2-(p-Trichlormethyl-benzoylmethylen)-3-ethyl-benzthiazolin
Vergleichsbeispiel 13: 2-(4-Methoxy-naphth-1-yl)-4,6-bis-trichlormethyl-s-triazin
Vergleichsbeispiel 14: 2-(4-Methoxy-styryl)-5-trichlormethyl-1,3,4-oxadiazol
Vergleichsbeispiel 15: 2-(4-Styrylphenyl)-4,6-bis-trichlormethyl-s-triazin (Verbindung Nr. 1 der EP-A 137 452)

Die Belichtung erfolgte mit der im Anwendungsbeispiel 1 beschriebenen Metallhalogenid-Lampe, wobei die Vorlage durch ein Kantenfilter, das nur für Strahlung oberhalb 400 nm durchlässig ist, abgedeckt wurde.

Alle Muster wurden 25 Sekunden lang belichtet und wie im Anwendungsbeispiel 1 angegeben weiterverarbeitet. Man erhielt folgende Ergebnisse:

Anwendungsbeispiel 11: 7 vollvernetzte Keilstufen,
Vergleichsbeispiel 12: 6 vollvernetzte Keilstufen, Stufe 7 teilvernetzt,
Vergleichsbeispiel 13: 4 vollvernetzte Keilstufen,
Vergleichsbeispiel 14: 1 vollvernetzte Keilstufe,
Vergleichsbeispiel 15: 2 vollvernetzte Keilstufen.

Daraus wird ersichtlich, daß der erfindungsgemäße Photoinitiator neben dem des Vergleichsbeispiels 12 oberhalb 400 nm am wirksamsten ist.

## Anwendungsbeispiel 16 und Vergleichsbeispiele 17 - 20

Die im Anwendungsbeispiel 1 angegebene Mischung wurde unter Ersatz der Verbindung 1 durch eine äquimolare Menge eines der nachstehend beschriebenen Photoinitiatoren auf den dort beschriebenen Träger aufgebracht.

Anwendungsbeispiel 16: Verbindung Nr. 2
Vergleichsbeispiel 17: 2-(p-Trichlormethyl-benzoylmethylen)-3-ethyl-benzthiazolin
Vergleichsbeispiel 18: 2-(4-Methoxy-naphth-1-yl)-4,6-bis-trichlor-methyl-s-triazin
Vergleichsbeispiel 19: 2-(4-Methoxy-styryl)-5-trichlormethyl-1,3,4-oxadiazol
Vergleichsbeispiel 20: 2-(4-Styrylphenyl)-4,6-bis-trichlormethyl-s-triazin

Die Belichtung erfolgte mit der in Beispiel 1 beschriebenen Metallhalogenid-Lampe, wobei die Vorlage durch ein Kantenfilter, das nur für Strahlung oberhalb 455 nm durchlässig ist, abgedeckt wurde.

Alle Muster wurden 40 Sekunden lang belichtet und wie im Anwendungsbeispiel 1 angegeben weiterverarbeitet. Man erhielt folgende Ergebnisse:

Anwendungsbeispiel 16: 3 vollvernetzte Keilstufen,
Vergleichsbeispiel 17: 0 vollvernetzte Keilstufen,
Vergleichsbeispiel 18: 0 vollvernetzte Keilstufen,
Vergleichsbeispiel 19: 0 vollvernetzte Keilstufen,
Vergleichsbeispiel 20: 0 vollvernetzte Keilstufen.

Daraus wird ersichtlich, daß der erfindungsgemäße Photoinitiator unter den vorgegebenen Bedingungen die einzige Verbindung ist, die eine Polymerisation bei Bestrahlung mit Wellenlängen oberhalb 455 nm auszulösen vermag.

Mit den nachfolgenden Anwendungsbeispielen soll die spektrale Empfindlichkeit erfindungsgemäßer und bekannter Photoinitiatoren belegt werden.

Anwendungsbeispiele 21 bis 31 und Vergleichsbeispiele 32 bis 34

Die im Anwendungsbeispiel 1 angegebene Mischung wurde jeweils unter Ersatz der Verbindung Nr. 1 durch eine äquimolare Menge eines der nachstehend beschriebenen Photoinitiatoren auf den dort beschriebenen Träger aufgebracht.

Die Proben wurden unter einer Lichtquelle, die im Bereich zwischen 300 und 700 nm praktisch konstant emittiert, durch ein Verlaufsfilter, das zwischen 400 und 600 nm kontinuierlich durchlässig ist, belichtet. Nach der Entwicklung mit der im Anwendungsbeispiel 1 angegebenen Entwicklerlösung wurde der durchgehärtete Bereich bestimmt (Spektrale Ansprechbarkeit).

Die Proben wurden weiterhin durch ein Kantenfilter, das oberhalb 455 nm durchlässig ist, wie im Beispiel 16 beschrieben, belichtet. Auch hier wurde mit der im Anwendungsbeispiel 1 beschriebenen Entwicklerlösung entwickelt.

In der folgenden Tabelle sind die vollvernetzten Keilstufen angegeben.

| Beispiel | Verb.-Nr. | Spektrale Ansprechbarkeit (nm) | Vernetzte Keilstufe |
|---|---|---|---|
| 21 | 1 | 430 - 530 | 2 - 3 |
| 22 | 6 | 430 - 530 | 2 - 3 |
| 23 | 10 | 430 - 530 | 2 - 3 |
| 24 | 13 | 460 - 480 | 1 |
| 25* | 17 | 430 - 570 | 4 |
| 26 | 21 | 440 - 540 | 5 |
| 27 | 23 | 410 - 580 | 9 |
| 28 | 24 | 420 - 580 | 10 |
| 29 | 25 | 470 - 520 | 2 |
| 30 | 33 | 500 - 550 | 3 |
| 31 | 36 | 480 - 550 | 4 - 5 |
| 32 V | 1) | 415 - 450 | 0 |
| 33 V | 2) | < 400 | 0 |
| 34 V | 3) | 400 - 420 | 0 |

* Der Photoinitiator konnte nicht vollständig in Lösung gebracht werden.
1) Verbindung XII der EP-A 135 348
2) Verbindung 1 der EP-A 137 452
3) 9-Phenylacridin

In den nachstehenden Anwendungsbeispielen 35 bis 49 werden quantitative Belichtungsergebnisse wiedergegeben, die durch Belichtung mit einem Argon-Ionen-Laser der Fa. Spectra-Physics erzielt wurden.

Anwendungsbeispiele 35 bis 41

Die im Anwendungsbeispiel 1 angegebene Mischung wurde unter Ersatz der Verbindung 1 durch eine äquimolare Menge eines der nachstehend beschriebenen Photoinitiatoren auf den dort beschriebenen Träger aufgebracht. Die Proben wurden mit Laserlicht der Wellenlängen 458, 476, 488, 504 und 514 nm bestrahlt. Die Entwicklung erfolgte wie im Anwendungsbeispiel 1 angegeben.

| Beispiel Nr. | Verb.-Nr. | Energiebedarf ($mJ/cm^2$) bei (nm) | | | | |
|---|---|---|---|---|---|---|
| | | 458 | 476 | 488 | 504 | 514 |
| 35 | 10 | 7 | 9 | 14 | 22 | 34 |
| 36 | 14 | 4 | 5 | 8 | 18 | 25 |
| 37 | 21 | 11,5 | 11 | 8 | 12 | 13 |
| 38 | 23 | 3 | 3 | 3 | 4 | 4 |
| 39 | 24 | 2 | 3 | 3 | 4 | 5 |
| 40 | 33 | 25 | 15 | 14 | 15 | 23 |
| 41 | 36 | 19 | 15 | 15 | 17 | 20 |

Aus diesen Beispielen geht hervor, daß die erfindungsgemäßen Photoinitiatoren im sichtbaren Bereich praxisgerecht ansprechen.

Anhand der folgenden Beispiele 42 bis 44 soll die hervorragende Lagerstabilität der erfindungsgemäßen Aufzeichnungsmaterialien illustriert werden.

Anwendungsbeispiel 42

Entsprechend Anwendungsbeispiel 1 wurden 5 lichtempfindliche Druckplatten hergestellt und unbelichtet eine, zwei, drei und vier Stunden in einem Umluftschrank auf 100 °C erwärmt. Nach Ablauf der Zeit wurden die Platten herausgenommen, abgekühlt, 15 Sekunden belichtet und wie im Anwendungsbeispiel 1 beschrieben weiterverarbeitet. Zum Vergleich wurde eine nicht erhitzte Platte belichtet und ebenso verarbeitet.

Während die eine, zwei und drei Stunden erhitzten Platten praktisch keinerlei Unterschied zum Vergleichsmuster zeigten, war der Halbtonstufenkeil bei der 4 Stunden erhitzten Platte um eine Stufe mehr vernetzt.

Dies zeigt die außergewöhnlich gute thermische Stabilität des erfindungsgemäßen Gemischs.

Anwendungsbeispiel 43

Entsprechend Anwendungsbeispiel 1 beschichtete Aluminiumplatten wurden unbelichtet in einem Wärmeschrank (Hotbox) bei einer Temperatur von 56 °C zwei, sechs und dreizehn Wochen aufbewahrt. Nach der jeweiligen Entnahme der Platten wurden diese, wie im Anwendungsbeispiel 1 beschrieben, weiterverarbeitet.

Die in der Hotbox gelagerten Platten zeigten auch nach 3 Monaten weder in der Kopie noch im Druck einen signifikanten Unterschied zu der Originalplatte des Beispiels 1.

Die Lagerstabilität bei erhöhter Temperatur ist demnach sehr gut.

Anwendungsbeispiel 44

Entsprechend Anwendungsbeispiel 1 beschichtete Aluminiumplatten wurden unbelichtet in einem Tropenschrank bei einer Temperatur von 42 °C und einer relativen Feuchtigkeit von 60 % zwei, sechs und dreizehn Wochen aufbewahrt. Nach der jeweiligen Entnahme der Platten wurden diese, wie im Anwendungsbeispiel 1 beschrieben, weiterverarbeitet.

Die im Tropenschrank gelagerten Platten zeigten auch nach 3 Monaten weder in der Kopie noch im Druck einen signifikanten Unterschied zu der Originalplatte des Beispiels 1.

Die Lagerstabilität unter Tropenbedingungen ist demnach sehr gut.

## Patentansprüche

1. Verbindungen der allgemeinen Formel I

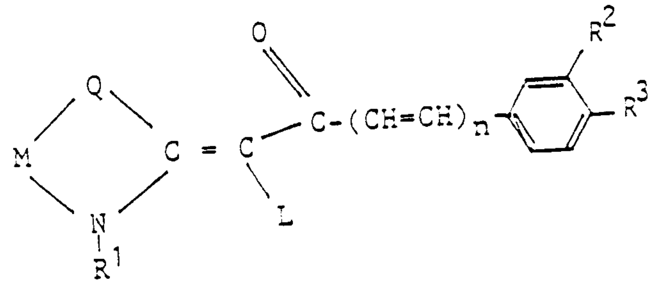

(I)

worin

L ein Wasserstoffatom oder einen Substituenten der Formel

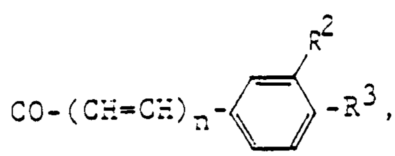

M einen 1,1-, 1,2- oder 1,3-Alkylenrest oder einen 1,2- oder 1,3-Alkenylenrest, die gegebenenfalls durch Halogenatome, Carboxy-, Carbonyl-, Alkoxy-, Alkyl- oder Arylgruppen substituiert sind; einen 1,2-Phenylenrest, der gegebenenfalls durch Halogenatome, Carboxy-, Sulfonsäure-, Nitro-, Cyan-, Carbonyl-, Alkyl-, Aryl-, Alkoxy-, Trifluormethyl- oder Alkoxycarbonylalkylgruppen substituiert ist, einen Pyridylen- oder Naphthylenrest,

Q ein Schwefel-, Selen- oder Sauerstoffatom, eine Dialkylmethylengruppe mit 3 bis 13 Kohlenstoffatomen, eine 1,2-Alkenylengruppe, die ggf. durch 1 oder 2 Alkyl- oder Arylreste, Chloratome, Alkoxygruppen oder Alkoxy-carbonylgruppen substituiert ist, einen gegebenenfalls durch Chloratome, Alkoxy- oder Alkoxycarbonylgruppen substituierten 1,2-Phenylenrest oder eine Gruppe $N-R^1$,

wobei M + Q zusammen 3 oder 4 Ringglieder bilden,

$R^1$ einen Alkyl- oder Alkoxyalkylrest mit 1 bis 10 Kohlenstoffatomen, einen Benzyl-, Chlorbenzyl-, Tolylmethyl-, Phenethyl- oder Cyclohexylrest,

$R^2$ und $R^3$ voneinander verschieden sind und entweder ein Wasserstoffatom oder eine 4,6-Bis-trichlormethyl-s-triazin-2-yl-gruppe bedeuten und

n = 0 oder 1 ist.

2. Verbindungen nach Anspruch 1, dadurch gekennzeichnet, daß n = 0 ist.

3. Verbindungen nach Anspruch 1, dadurch gekennzeichnet, daß $R^2$ ein Wasserstoffatom ist.

4. Verbindungen nach Anspruch 1, dadurch gekennzeichnet, daß Q ein Schwefel- oder Selenatom ist.

5. Lichtempfindliches Gemisch, enthaltend eine lichtempfindliche organische Verbindung (a) mit mindestens einem 4,6-Bis-trichlormethyl-s-triazin-2-ylsubstituenten und eine Verbindung (b), die mit dem Lichtreaktionsprodukt der Verbindung (a) unter Ausbildung eines Produktes zu reagieren vermag, das eine von (b) unterschiedliche Lichtabsorption, Klebrigkeit oder Löslichkeit in einem Entwickler aufweist, dadurch gekennzeichnet, daß die Verbindung (a) eine Verbindung gemäß Anspruch 1 ist.

6. Lichtempfindliches Gemisch nach Anspruch 5, dadurch gekennzeichnet, daß die Verbindung (b) eine ethylenisch ungesättigte Verbindung ist, die eine durch freie Radikale ausgelöste Polymerisation einzugehen vermag.

7. Lichtempfindliches Gemisch nach Anspruch 5, dadurch gekennzeichnet, daß die Verbindung (b) mindestens eine durch Säure spaltbare C-O-C-Bindung enthält.

8. Lichtempfindliches Gemisch nach Anspruch 5, dadurch gekennzeichnet, daß die Verbindung (b) durch Säure zur kationischen Polymerisation angeregt zu werden vermag.

9. Lichtempfindliches Gemisch nach Anspruch 5, dadurch gekennzeichnet, daß die Verbindung (b) durch Säure vernetzbar ist.

10. Lichtempfindliches Gemisch nach Anspruch 5, dadurch gekennzeichnet, daß die Verbindung (b) durch Einwirkung von Säure ihren Farbton ändert.

11. Lichtempfindliches Gemisch nach Anspruch 5, dadurch gekennzeichnet, daß es die Verbindung der Formel I in einer Menge von 0,1 bis 15 Gew.-%, bezogen auf seine nichtflüchtigen Bestandteile, enthält.

12. Lichtempfindliches Gemisch nach Anspruch 5, dadurch gekennzeichnet, daß es zusätzlich ein wasserunlösliches polymeres Bindemittel enthält.

13. Lichtempfindliches Gemisch nach Anspruch 12, dadurch gekennzeichnet, daß das Bindemittel in wäßrig-alkalischen Lösungen löslich ist.

14. Verfahren zur Herstellung einer Verbindung der Formel I gemäß Anspruch 1, dadurch gekennzeichnet, daß man eine Verbindung der Formel II

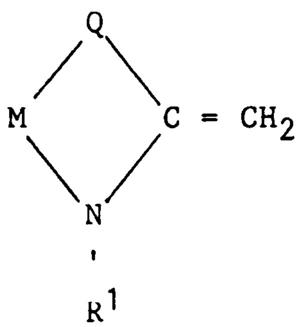

(II)

oder deren Iminiumsalz der Formel III

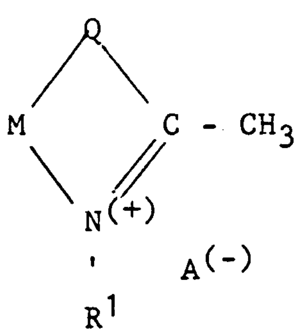

(III)

worin $A^{(-)}$ das Anion des Iminiumsalzes bedeutet, mit einem Carbonsäurehalogenid der Formel IV

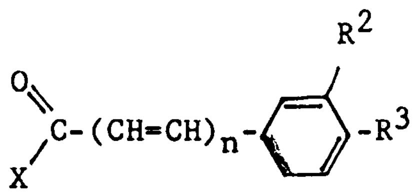

(IV)

umsetzt, wobei X ein Halogenatom ist und die Symbole L, M, Q, $R^1$, $R^2$, $R^3$ und n die im Anspruch 1 angegebene Bedeutung haben.

**Claims**

**1.** Compounds of the general formula I

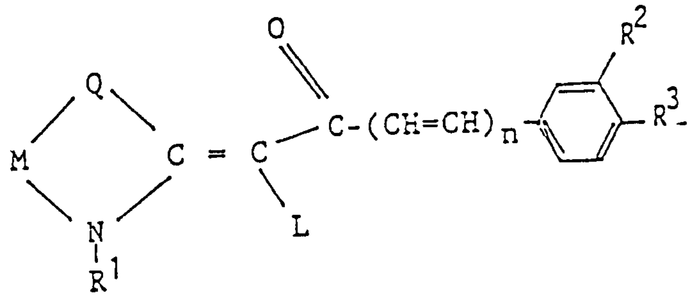

(I)

wherein

| | |
|---|---|
| L | denotes a hydrogen atom or a substituent of the formula |

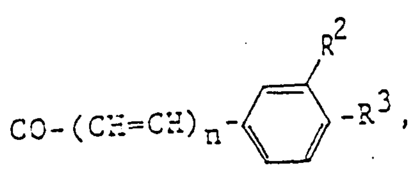

| | |
|---|---|
| M | denotes a 1,1-, 1,2- or 1,3-alkylene radical or a 1,2- or 1,3-alkenylene radical, which are optionally substituted by halogen atoms, carboxy, carbonyl, alkoxy, alkyl or aryl groups; a 1,2-phenylene radical which is optionally substituted by halogen atoms, carboxy, sulfo, nitro, cyano, carbonyl, alkyl, aryl, alkoxy, trifluoromethyl or alkoxycarbonylalkyl groups; a pyridylene or naphthylene radical, |
| Q | denotes a sulfur, selenium or oxygen atom, a dialkylmethylene group having from 3 to 13 carbon atoms, a 1,2-alkenylene group which is optionally substituted by 1 or 2 alkyl or aryl radicals, chlorine atoms, alkoxy groups or alkoxycarbonyl groups, a 1,2-phenylene radical which is optionally substituted by chlorine atoms, alkoxy or alkoxycarbonyl groups, or a group N-$R^1$, |
| $R^1$ | denotes an alkyl or alkoxyalkyl radical having from 1 to 10 carbon atoms, a benzyl, chlorobenzyl, tolylmethyl, phenethyl or cyclohexyyl radical, |
| $R^2$ and $R^3$ | differ from one another and either denote a hydrogen atom or a 4,6-bis-trichloromethyl-s-triazin-2-yl-group, and |
| n | = 0 or 1. |

**2.** Compounds as claimed in claim 1, characterized in that n = 0.

**3.** Compounds as claimed in claim 1, characterized in that, $R^2$ is a hydrogen atom.

4. Compounds as claimed in claim 1, characterized in that, Q is a sulfur or selenium atom.

5. A photosensitive composition, containing a photosensitive organic compound (a) having at least one 4,6-bis-trichloromethyl-s-triazin-2-yl substituent and a compound (b) which is capable or reacting with the photoreaction product of compound (a), forming a product, of which the light absorption, tackiness or solubility in a developer differs from that of (b), characterized in that compound (a) is a compound as claimed in claim 1.

6. The photosensitive composition as claimed in claim 5, characterized in that compound (b) is an ethylenically unsaturated compound which is capable of undergoing a polymerization initiated by free radicals.

7. The photosensitive composition as claimed in claim 5, characterized in that compound (b) contains at least one C-O-C bond which can be split by an acid.

8. The photosensitive composition as claimed in claim 5, characterized in that compound (b) can be induced to cationic polymerization by an acid.

9. The photosensitive composition as claimed in claim 5, characterized in that compound (b) can be crosslinked by an acid.

10. The photosensitive composition as claimed in claim 5, characterized in that compound (b) changes its color shade under the action of an acid.

11. The photosensitive composition as claimed in claim 5, characterized in that it contains the compound of the formula I in a quantity from 0.1 to 15 % by weight, based on its non-volatile constituents.

12. The photosensitive composition as claimed in claim 5, characterized in that it additionally contains a water-insoluble polymeric binder.

13. The photosensitive composition as claimed in claim 12, characterized in that the binder is soluble in aqueous-alkaline solutions.

14. A process for preparing a compound of the formula I as claimed in claim 1, characterized in that a compound of the formula II

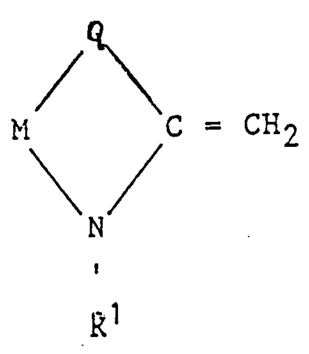

(II)

or its iminium salt of the formula III

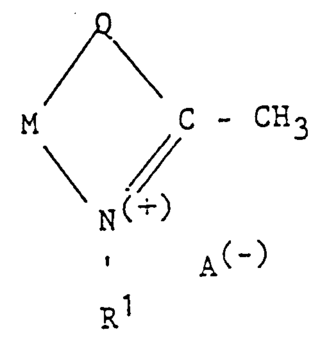

(III)

in which $A^{(-)}$ is the anion of the iminium salt, is reacted with a carboxylic acid halide of the formula IV

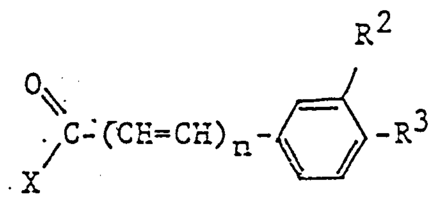

(IV)

X being a halogen atom and the symbols L, M, Q, $R^1$, $R^2$, $R^3$ and n being as defined in claim 1.

## Revendications

**1.** Composés de formule générale I

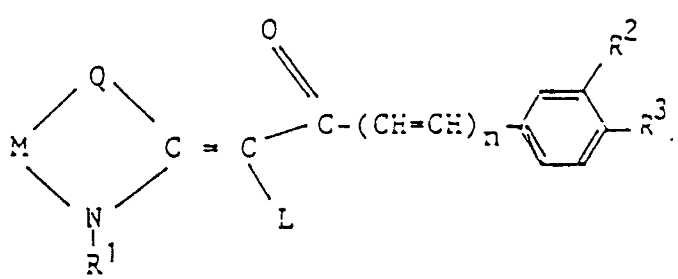

(I)

dans laquelle

L est un atome d'hydrogène ou un substituant de formule

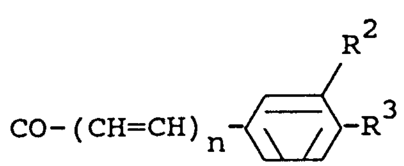

M est un radical 1,1-, 1,2- ou 1,3-alkylène ou un radical 1,2- ou 1,3-alcénylène, qui sont éventuellement substitués par des atomes d'halogène, des groupes carboxy, carbonyle, alcoxy, alkyle ou aryle; un radical 1,2-phénylène qui est éventuellement substitué par des atomes d'halogène, des groupes carboxy, sulfo, nitro, cyano, carbonyle, alkyle, aryle, alcoxy, trifluorométhyle ou alcoxycarbonylalkyle; un radical pyridylène ou naphtylène;

Q est un atome de soufre, de sélénium ou d'oxygène, un groupe dialkylméthylène ayant de 3 à 13 atomes de carbone, un groupe 1,2-alcénylène qui est éventuellement substitué par 1 ou 2 atomes de chlore ou radicaux alkyle ou aryle, alcoxy ou alcoxycarbonyle, un groupe 1,2-phénylène éventuellement substitué par des atomes de chlore, des groupes alcoxy ou alcoxycarbonyle, ou un radical N-$R^1$;

M + Q formant ensemble 3 ou 4 chaînons de cycle;

| | |
|---|---|
| $R^1$ | est un radical alkyle ou alcoxyalkyle ayant de 1 à 10 atomes de carbone, un radical benzyle, chlorobenzyle, tolylméthyle, phénéthyle ou cyclohexyle; |
| $R^2$ et $R^3$ | sont différents l'un de l'autre et représentent soit un atome d'hydrogène, soit le groupe 4,6-bis-trichlorométhyl-s-triazine-2-yle; et |
| n | est 0 ou 1. |

2. Composés selon la revendication 1, caractérisés en ce que n = 0.

3. Composés selon la revendication 1, caractérisés en ce que $R^2$ est un atome d'hydrogène.

4. Composés selon la revendication 1, caractérisés en ce que Q est un atome de soufre ou de sélénium.

5. Composition photosensible, contenant un composé (a) organique photosensible comportant au moins un substituant 4,6-bis-trichlorométhyl-s-triazine-2-yle, et un composé (b) susceptible de réagir avec le produit de photoréaction du composé (a) avec formation d'un produit qui présente une absorption de la lumière, adhésivité ou solubilité dans un révélateur différentes de (b), caractérisée en ce que le composé (a) est un composé selon la revendication 1.

6. Composition photosensible selon la revendication 5, caractérisée en ce que le composé (b) est un composé à insaturation éthylénique, qui est susceptible de subir une polymérisation déclenchée par des radicaux libres.

7. Composition photosensible selon la revendication 5, caractérisée en ce que le composé (b) comporte au moins une liaison C-O-C pouvant être rompue par un acide.

8. Composition photosensible selon la revendication 5, caractérisée en ce que le composé (b) est susceptible d'être induit à la polymérisation cationique par un acide.

9. Composition photosensible selon la revendication 5, caractérisée en ce que le composé (b) est réticulable par un acide.

10. Composition photosensible selon la revendication 5, caractérisée en ce que la nuance du composé (b) change sous l'effet d'un acide.

11. Composition photosensible selon la revendication 5, caractérisée en ce qu'elle contient le composé de formule I en une proportion de 0,1 à 15 % en poids, par rapport à ses composants non volatils.

12. Composition photosensible selon la revendication 5, caractérisée en ce qu'elle contient en outre un liant polymère insoluble dans l'eau.

13. Composition photosensible selon la revendication 12, caractérisée en ce que le liant est soluble dans des solutions aqueuses-alcalines.

14. Procédé pour la préparation d'un composé de formule I selon la revendication 1, caractérisé en ce que l'on fait réagir un composé de formule II

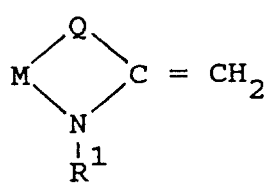

(II)

ou son sel d'iminium de formule III

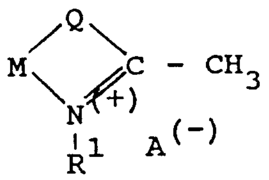

(III)

dans laquelle $A^{(-)}$ représente l'anion du sel d'iminium, avec un halogénure d'acide carboxylique de formule IV

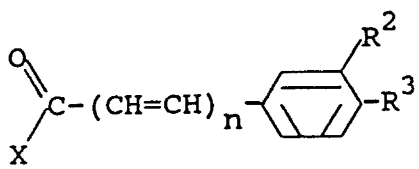

(IV)

X étant un atome d'halogène et les symboles L, M, Q, $R^1$, $R^2$, $R^3$ et n ayant les significations données dans la revendication 1.